# EUROPEAN PATENT APPLICATION

(11) **EP 4 506 367 A1**
(43) Date of publication of application: **12.02.2025**
(21) Application number: 23778495.4
(22) Date of filing: 31.03.2023
(51) Int. Cl.: C07K 16/46, C07K 16/22, C12N 15/62, C12N 15/13, C12N 15/63, A61K 39/395, A61P 35/00, A61P 19/02, A61P 27/02

(54) **AAV VECTOR ENCODING ANTI-VEGF-A AND ANG-2 BISPECIFIC ANTIBODY**

(30) Priority: 02.04.2022 CN 202210356939
(71) Applicant: Starrygene Therapeutics Co., Ltd., Hefei, Anhui 230031 (CN)
(72) Inventor: CAI, Yuan, Hefei, Anhui 230031 (CN); MA, Zhen, Hefei, Anhui 230031 (CN); ZHOU, Peipei, Hefei, Anhui 230031 (CN); ZHANG, Mingliang, Hefei, Anhui 230031 (CN); ZHAO, Jin, Hefei, Anhui 230031 (CN)
(74) Representative: J A Kemp LLP
(86) International application number: PCT/CN2023/085610
(87) International publication number: WO 2023/186131

(57) **Abstract**

Provided are a bispecific antibody, an encoding nucleic acid thereof, an AAV virus vector containing the nucleic acid, and a virus particle. Further provided is a pharmaceutical composition containing the bispecific antibody, the encoding nucleic acid, the AAV virus vector, or the virus particle. The bispecific antibody comprises binding domains specific to VEGF-A and ANG-2, and can be used for treating wet age-related macular degeneration or diabetic macular edema of a subject.

## Description

### TECHNICAL FIELD

The present invention relates to the field of immunology and gene delivery. More particularly, the present application relates to compositions, systems, and methods for producing proteins of interest, such as antibodies.

### BACKGROUND

Age-related macular degeneration (AMD) is a group of age-related macular diseases induced by various factors. Their common features are the lesions of the macular retina and retinal pigment epithelium and choroid, which cause visual dysfunction and progressive decrease in central vision of patients. The prevalence of AMD in China and even the world is increasing with age, and AMD is one of the main causes of irreversible vision impairment in people over 50 years old. As the population ages, AMD patients are expected to reach 288 million by 2040. There is no unified standard for the clinical classification of AMD. At present, both domestic and foreign countries tend to stage first and then classify, and the late stage is divided into 2 types, which are dry (atrophic) and neovascular (exudative, wet), among which, neovascular age-related macular degeneration (nAMD) , also called wet age-related macular degeneration (wAMD), is a main clinical type causing vision loss, and its main feature is that choroidal neovascularization (CNV) appears in the macular area, thereby causing bleeding and exudation in the macular area. Diabetic macular edema (DME) is currently one of important causes of blindness in western developed countries, and with the improvement of living standards of people in China and the aging of the population, the prevalence rate of DME has gradually increased, which seriously affects visual function and life quality of patients.

Vascular endothelial growth factor (VEGF) is one of the most important factors in the pathogenesis of wAMD and DME, and can specifically act on vascular endothelial cells, promote the proliferation of vascular endothelial cells, induce the formation of neovessels, and increase vascular leakage. At present, it has been found that blocking the expression of VEGF can induce vascular remodeling and promote the regression of immature neovascularization. Therfore, VEGF is a promising therapeutic target for wAMD and DME. Since the application of anti-VEGF drugs represented by Ranibizumab in 2006, anti-VEGF drugs have been widely used to treat wAMD and DME. In recent 10 years, irreversible blindness caused by wAMD and DME has been greatly reduced due to the simultaneous application of multiple anti-VEGF therapies.

Although there have been great progress and breakthroughs VEGF-targeting drugs in clinicals, which can significantly reduce the degree of vascular leakage and edema, improve vision, and have not found serious complications, these protein drugs injected into the body are rapidly eliminated through metabolism, thus multiple intraocular injections are required to maintain the therapeutic effects. Long-term treatment increases the economic burden of patients, repeated injection increases the pain and the possibility of adverse reactions of patients, some degree of vision loss occurs when changing from conventional administration to low-frequency administration, and some patients are prone to relapse after treatment. In response to the limitations of existing therapies for wAMD and DME, there is a need in the art for more economical, longer lasting and more effective treatment strategies.

The present invention aims to provide an anti-VEGF-A and anti-ANG-2 gene therapy.

### SUMMARY

The present invention provides a bispecific antibody comprising anti-VEGF-A and anti-ANG-2 binding domain, which is preferably a tandem single chain antibody molecule (tandem scFv, ta-scFv), wherein the heavy chain variable regions (VH) and the light chain variable regions (VL) of the bispecific antibody are arranged from N-terminus to C-terminus in the following order:
1) VH_{anti-VEGF-A}-VL_{anti-VEGF-A}-VH_{anti-ANG-2}-VL_{anti-ANG-2};
2) VL_{anti-ANG-2}-VH_{anti-VEGF-A}-VL_{anti-VEGF-A}-VH_{anti-ANG-2}; or
3) VL_{anti-VEGF-A}-VH_{anti-ANG-2}-VL_{anti-ANG-2}-VH_{anti-VEGF-A};

wherein the VH_{anti-VEGF-A} comprises a CDR1 as depicted in SEQ ID NO: 1, a CDR2 as depicted in SEQ ID NO: 2, and a CDR3 as depicted in SEQ ID NO: 3;
the VL_{anti-VEGF-A} comprises a CDR1 as depicted in SEQ ID NO: 4, a CDR2 as depicted in SEQ ID NO: 5, and a CDR3 as depicted in SEQ ID NO: 6 or SEQ ID NO: 47;
the VH_{anti-ANG-2} comprises a CDR1 as depicted in SEQ ID NO: 7, a CDR2 as depicted in SEQ ID NO: 8, and a CDR3 as depicted in SEQ ID NO: 9;
the VL_{anti-ANG-2} comprises a CDR1 as depicted in SEQ ID NO: 10, a CDR2 as depicted in SEQ ID NO: 11, and a CDR3 as depicted in SEQ ID NO: 12.

In one embodiment, the VH_{anti-VEGF-A} comprises a sequence as depicted in SEQ ID NO: 13 or a sequence that is 70%, 80%, 90%, 95% or 99% identical to the sequence of SEQ ID NO: 13;
the VL_{anti-VEGF-A} comprises a sequence as depicted in SEQ ID NO: 14 or a sequence that is 70%, 80%, 90%, 95% or 99% identical to the sequence of SEQ ID NO: 14 or SEQ ID NO: 48;
the VH_{anti-ANG-2} comprises a sequence as depicted in SEQ ID NO: 15 or a sequence that is 70%, 80%, 90%, 95% or 99% identical to the sequence of SEQ ID NO: 15;
the VL_{anti-ANG-2} comprises a sequence as depicted in SEQ ID NO: 16 or a sequence that is 70%, 80%, 90%, 95% or 99% identical to the sequence of SEQ ID NO: 16.

In one embodiment, the antibody comprises a sequence as depicted in SEQ ID NO: 17, 18, 19, 49, 51 or 53 or a sequence that is 70%, 80%, 90%, 95% or 99% identical to the sequence of SEQ ID NO: 17, 18, 19, 49, 51 or 53.

In one embodiment, the N-terminus of the bispecific antibody construct comprises a signal peptide sequence or a tag sequence, preferably the signal peptide sequence is a CD5-sp signal peptide comprising a sequence as depicted in SEQ ID NO: 23.

In one embodiment, the antibody comprises a sequence as depicted in SEQ ID NO: 20, 21, 22, 50, 52 or 54 or a sequence that is 70%, 80%, 90%, 95% or 99% identical to the sequence of SEQ ID NO: 20, 21, 22, 50, 52 or 54.

In one embodiment, the heavy chain variable region (VH) and the light chain variable region (VL) are operably linked by (G₄S)ₙ, where n is an integer greater than 1, preferably any integer between 1 and 4, such as G₄S, (G₄S)₂, (G₄S)₃ or (G₄S)₄, for example,
1)VH_{anti-VEGF-A} - (G₄S)_{m+X} - VL_{anti-VEGF-A} - (G₄S)ₘ - VH_{anti-ANG-2} - (G₄S)_{m+X} - VL_{anti-ANG-2}; wherein m ≥ 1, X ≥ 2, preferably VH_{anti-VEGF-A} - (G₄S)₃ - VL_{anti-VEGF-A} - GaS - VH_{anti-ANG-2} - (G₄S)₃ - VL_{anti-ANG-2};
2)VL_{anti-ANG-2} - (G₄S)ₘ - VH_{anti-VEGF-A} - (G₄S)_{m+X} - VL_{anti-VEGF-A} - (G₄S)ₘ - VH_{anti-ANG-2}, wherein m ≥ 1, X ≥ 2, preferably VL_{anti-ANG-2} - G₄S - VH_{anti-VEGF-A} - (G₄S)₃ - VL_{anti-VEGF-A} - GaS - VH_{anti-ANG-2}; or
3)VL_{anti-VEGF-A} - (G₄S)ₘ - VH_{anti-ANG-2} - (G₄S)_{m+X} - VL_{anti-ANG-2} - (G₄S)ₘ - VH_{anti-VEGF-A}, wherein m ≥ 1, X ≥ 2, preferably VL_{anti-VEGF-A} - GaS - VH_{anti-ANG-2} - (G₄S)₃ - VL_{anti-ANG-2} - G₄S - VH_{anti-VEGF-A}.

Furthermore, the present invention provides a nucleic acid sequence encoding the bispecific antibody as described above.

The present invention also provides a vector comprising a nucleic acid sequence encoding the bispecific antibody.

In one embodiment, the vector is preferably AAV virus.

In one embodiment, the AAV virus vector further comprises: a 5' ITR and a 3' ITR, a promoter, and a polyA sequence.

In another aspect, the present invention provides an AAV virus particle, comprising any of the AAV virus vectors as described above and a capsid protein, preferably wherein the serotype of the capsid protein is AAV1, AAV2, AAV4, AAV5, AAV6, AAV7, AAV8 or AAV9.

The present invention provides a pharmaceutical composition comprising at least one of the bispecific antibody, nucleic acid sequence, vector such as AAV virus vector as described above and AAV virus particle as described above, and a pharmaceutically acceptable carrier.

The present invention provides a use of the bispecific antibody, nucleic acid sequence, vector such as AAV virus vector, AAV particle as described above, or pharmaceutical compositions as described above in the preparation of a medicament for treating or preventing cancer, intraocular neovascularization syndrome, rheumatoid arthritis, psoriasis, proliferative retinopathy, age-related macular degeneration or diabetic macular edema.

In one embodiment, the age-related macular degeneration is wet age-related macular degeneration.

In a specific embodiment, the medicament is administered by intravitreal or subretinal injection.

### BRIEF DESCRIPTION OF THE DRAWINGS

FIG. 1 shows schematic structural diagrams of XMVA01, XMVA04, and XMVA09.
FIG. 2 shows vector information of pAAV9-XMVA09, ssAAV-XMVA09.
FIG. 3 shows expression levels of VEGF165 neutralizing proteins in cells after transfection by pAAV9-XMVA01, pAAV9-XMVA04 and pAAV9-XMVA09 vectors.
FIG. 4 shows effect of expressing XMVA01, XMVA04 and XMVA09 in cells on the proliferation of human retinal microvascular endothelial cells (HRMECs) under ECGS stimulation.
FIGs. 5, 6 show the effects of expressing XMVA01, XMVA04 and XMVA09 in cells on the tube formation of HRMECs.
FIG. 7 shows a schematic structural diagram of XMVA10.
FIG. 8 shows a schematic structural diagram of XMVA11.
FIG. 9 shows a schematic structural diagram of XMVA13.
FIG. 10 shows a schematic structural diagram of XMVA14.
FIG. 11 shows a schematic structural diagram of XMVA15.
FIG. 12 shows a fluorescein fundus angiography (FFA) images of laser-induced wAMD model mice after injection of AAV-XMVA09.
FIG. 13 shows percentage of grade 3 light spots and mean score of leakage light spots after laser-induced wAMD model mice were injected with AAV-XMVA09.
FIG. 14 shows the changes of grade IV light spots number and fluorescence leakage area in the laser-induced wAMD model rhesus monkey control group.
FIG. 15 shows the changes of grade IV light spots number and fluorescence leakage area in the laser-induced wAMD model rhesus monkey injected with AAV-XMVA09.
FIG. 16 shows the changes in retinal thickness of grade IV light spots in the laser-induced wAMD model rhesus monkey control group.
FIG. 17 shows the changes in retinal thickness of grade IV light spots in the laser-induced wAMD model rhesus monkey injected with AAV-XMVA09.
FIG. 18 shows the changes in permeability of high glucose induced HRMECs.
FIG. 19 shows the regulatory effect of XMVA09 on the permeability of HRMECs based on VE-cadherin indicator.
FIG. 20 shows regulatory effect of XMVA09 on the permeability of HRMECs based on the biotin-avidin system indicator.
FIG. 21 shows monitoring analysis of fasting blood glucose and body weight in diabetic model mice.
FIG. 22 shows images of retinal vascular leakage in diabetic model mouse.
FIG. 23 shows the changes in retinal vascular leakage area in diabetic model mice after injection of AAV-XMVA09.

### DETAILED DESCRIPTION

The present invention will be described in detail below according to embodiments and in conjunction with the accompanying drawings. The above aspects of the invention and other aspects of the invention will be apparent in the following detailed description. The scope of the present invention is not limited to the following examples.

The antibodies in the present invention are multispecific, and can be humanized, single-chain, chimeric, synthetic, recombinant, heterozygous, mutant, and grafted andtibodies; the antibody format in the present invention is a scFv spliced by antibody light chain variable regions (VL) and antibody heavy chain variable regions (VH), and the antibody can bind to two different antigens, such as VEGF-A and ANG-2.

The antibody light chain variable region (VL) and antibody heavy chain variable region (VH) can be further subdivided into hypervariable regions called complementarity determining regions (CDRs), and interspersed more conserved regions called framework regions (FWR). The CDRs of the antibodies and antigen-binding fragments disclosed herein are defined or identified by Kabat numbering. In one embodiment, each VH and VL generally includes 3 CDRs and 4 FWRs arranged in the following order from the amino end to the carboxy end: FWR1, CDR1, FWR2, CDR2, FWR3, CDR3, FWR4. The CDRs of the antibodies and antigen-binding fragments disclosed herein are defined or recognized by Kabat numbering.

As used herein, "vector" refers to a vector comprising a recombinant polynucleotide comprising an expression control sequence operably linked to a nucleotide sequence to be expressed.

The experimental methods in the following examples, unless otherwise specified, are all conventional methods.

### EXAMPLES

### Example 1. Construction of plasmid vector

### Construction of AAV vector plasmid expressing anti-VEGF-A/anti-ANG-2 gene

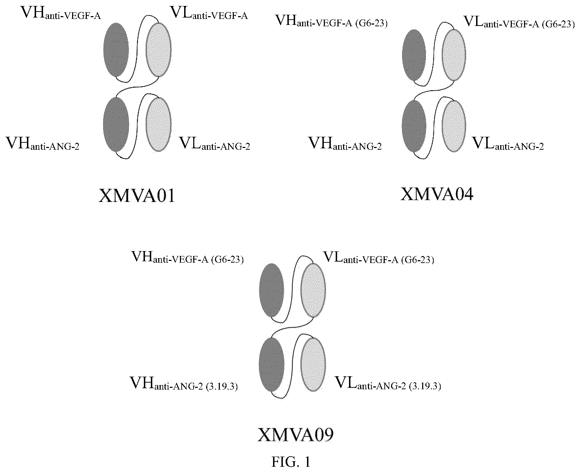

The VH amino acid sequence and the VL amino acid sequence of anti-VEGF, the VH amino acid sequence and the VL amino acid sequence of anti-ANG-2 were linked with (G₄S)₃, G₄S, and (G₄S)₃ peptide linkers, respectively, with a secretory signal peptide CD5-sp nucleotide sequence added to the N-terminus to form an open reading frame with a structure of CD5-sp-VH_{anti-VEGF-A} - (GaS)3 - VL_{anti-VEGF-A} - GaS - VH_{anti-ANG-2} - (G₄S)₃-VL_{anti-ANG-2}; the nucleotide sequences were designed according to human codon preference, with a BamH I cleavage site introduced at the 5' terminus, and an EcoR V cleavage site introduced at the 3' terminus, constructed according to the respective sequences in Table 1 and Table 2, and were named as XMVA01, XMVA04 and XMVA09 (the full-length genes were synthesized by Nanjing GenScript Biotech Corporation). The schematic diagrams of their structures are shown in FIG. 1.

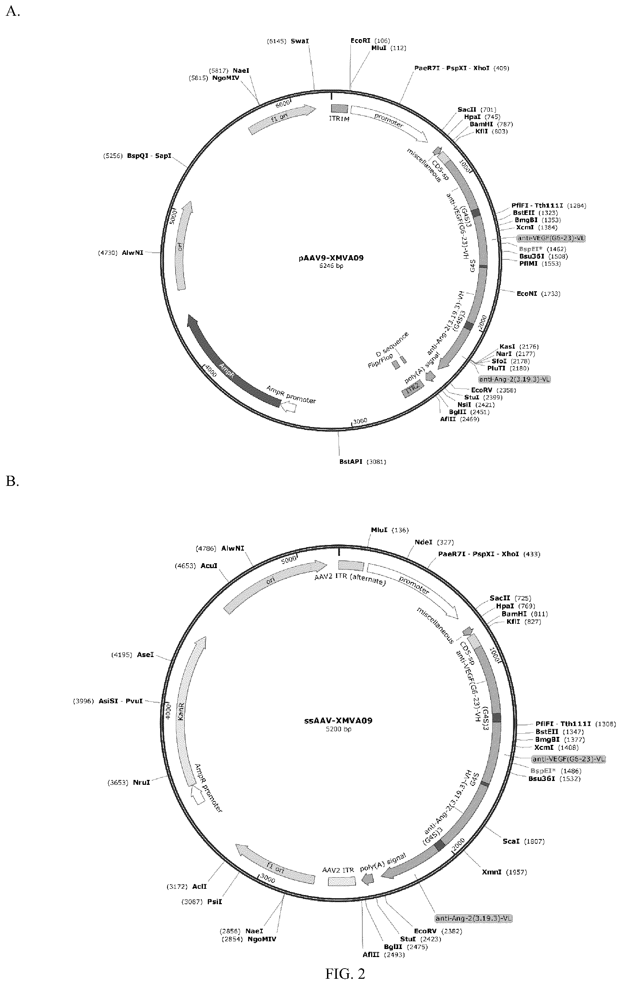

The above constructs and pAAV9neo-CAG plasmid were double digested with BamH I/EcoR V, and pAAV9-XMVA01, pAAV9-XMVA04 and pAAV9-XMVA09 vectors were constructed respectively by conventional molecular biology operations such as ligation, transformation, cloning screening and identification; the information of pAAV9-XMVA09 vector was shown in FIG. 2A. High-quality plasmid DNA was obtained for later use by using an endotoxin-free plasmid extraction kit (MN).

**Table 1. Sequence information of XMVA09**

| Product Number | Sequence information | amino acid sequence | | DNA sequence | |
|---|---|---|---|---|---|
| | VH _{anti-VEGF-A}(G6-23)-CDR1 | DYWIH | SEQ ID NO: 1 | gactactggattcac | SEQ ID NO: 24 |
| | VH _{anti-VEGF-} | GITPAGGYTYYADS | SEQ ID | ggaatcaccccagcaggaggctacacatactatgccgacagcgt | SEQ ID |
| | _{A}(G6-23)-CDR2 | VKG | NO: 2 | gaagggc | NO: 25 |
| | VH _{anti-VEGF-A}(G6-23)-CDR3 | FVFFLPYAMDY | SEQ ID NO: 3 | ttcgtgttcttcctgccatacgccatggattat | SEQ ID NO: 26 |
| | VH _{anti-VEGF-A}(G6-23) | EVQLVESGGGLVQ PGGSLRLSCAASGF TISDYWIHWVRQA PGKGLEWVAGITP AGGYTYYADSVKG RFTISADTSKNTAY LQMNSLRAEDTAV YYCARFVFFLPYA MDYWGQGTLVTV SS | SEQ ID NO: 13 | gaggtgcagctggtggagagcggaggaggactggtgcagcca ggaggctccctgcggctgtcttgcgccgccagcggctttaccatct ccgactactggattcactgggtgagacaggcacctggcaaggga ctggagtgggtggcaggaatcaccccagcaggaggctacacata ctatgccgacagcgtgaagggccggttcaccatctccgccgatac ctctaagaacacagcctatctgcagatgaactccctgcgggccga ggacacagccgtgtactattgcgccagattcgtgttcttcctgccat acgccatggattattggggccagggcaccctggtgacagtgagct cc | SEQ ID NO : 27 |
| | VL _{anti-VEGF-A}(G6-23)-CDR1 | RASQDVSTAVA | SEQ ID NO: 4 | agagcaagccaggacgtgagcaccgcagtggca | SEQ ID NO: 28 |
| | VL _{anti-VEGF-A}(G6-23)-CDR2 | SASFLYS | SEQ ID NO: 5 | tccgcctctttcctgtattct | SEQ ID NO: 29 |
| | VL _{anti-VEGF-A}(G6-23)-CDR3 | KQGYANPWT | SEQ ID NO: 6 | aagcagggctatgccaatccatggacc | SEQ ID NO: 30 |
| | VL_{anti-VEGF-A}(G6-23) | DIQMTQSPSSLSAS VGDRVTITCRASQ DVSTAVAWYQQKP GKAPKLLIYSASFL YSGVPSRFSGSGSG TDFTLTISSLQPEDF ATYYCKQGYANP WTFGQGTKVEIKR | SEQ ID NO: 14 | gatattcagatgacacagtccccatctagcctgtctgccagcgtgg gcgacagggtgaccatcacatgtagagcaagccaggacgtgag caccgcagtggcatggtaccagcagaagcctggcaaggcccca aagctgctgatctactccgcctctttcctgtattctggcgtgccaagc aggtttagcgggtccggatctggaaccgacttcaccctgacaatct cctctctgcagcctgaggattttgccacatactattgcaagcaggg ctatgccaatccatggaccttcggccagggcacaaaggtggagat caagagg | SEQ ID NO: 31 |
| | VH_{anti-ANG-2}(3.19.3)-CDR1 | NYGMH | SEQ ID NO: 7 | aactatggaatgcac | SEQ ID NO :32 |
| | VH_{anti-ANG-2}(3.19.3)-CDR2 | VISHDGNNKYYVD SVKG | SEQ ID NO: 8 | gtgatcagccacgacggcaacaacaagtactacgtggacagcgt gaagggc | SEQ ID NO: 33 |
| XMVA09 | VH_{anti-ANG-2}(3.19.3)-CDR3 | EGIDFWSGLNWFD P | SEQ ID NO: 9 | gagggcatcgacttttggagcggcctgaattggttcgaccct | SEQ ID NO: 34 |
| | VH_{anti-ANG-2}(3.19.3) | QVQLVESGGGVVQ PGRSLRLSCAASGF TFTNYGMHWGRQ APGKGLEWVAVIS HDGNNKYYVDSV KGRFTISRDNSKNT LYLQMNSLRAEDT AVYYCAREGIDFW SGLNWFDPWGQG TLVTVSSA | SEQ ID NO :15 | caggtgcagctggttgaatctggtggcggagttgtgcagcctggc agaagcctgagactgtcttgtgccgccagcggcttcaccttcacca actatggaatgcactggggcagacaggcccctggcaaaggactt gaatgggtcgccgtgatcagccacgacggcaacaacaagtacta cgtggacagcgtgaagggcagattcaccatcagccgggacaac agcaagaacaccctgtacctgcagatgaacagcctgagagccga ggacaccgccgtgtactattgtgccagagagggcatcgacttttg gagcggcctgaattggttcgacccttggggacagggcaccctgg ttacagtttcttctgcc | SEQ ID NO: 35 |
| | VL_{anti-ANG-2}(3.19.3)-CDR1 | RASQSITGSYLA | SEQ ID NO : 10 | agagccagccagagcatcaccggctcttacctggct | SEQ ID NO: 36 |
| | VL_{anti-ANG-2}(3.19.3)-CDR2 | GASSWAT | SEQ ID NO: 11 | ggcgcctcttcttgggccacc | SEQ ID NO: 37 |
| | VL_{anti-ANG-2}(3.19.3)-CDR3 | QQYSSSPIT | SEQ ID NO : 12 | cagcagtacagcagcagccccatcacc | SEQ ID NO: 38 |
| | VL _{anti-ANG-2}(3.19.3) | EIVLTQSPGTLSLSP GERATLSCRASQSI TGSYLAWYQQKPG QAPRLLICGASSWA TGIPDRFSGSGSGT DFTLTISRLEPEDFA VYYCQQYSSSPITF | SEQ ID NO: 16 | gagatcgtgctgacacagagccctggcacactgtcactgtctcca ggcgaaagagccacactgagctgtagagccagccagagcatca ccggctcttacctggcttggtatcagcagaagcctggacaggccc ctagactgctgatttgtggcgcctcttcttgggccaccggcattccc gatagattttctggcagcggctccggcaccgacttcaccctgacaa tcagcagactggaacccgaggacttcgccgtgtactactgccagc agtacagcagcagccccatcacctttggccagggcacaagactg | SEQ ID NO: 39 |
| | | GQGTRLEIKR | | gaaatcaagcgg | |
| | VH _{anti-VEGF-A}- (G₄S)₃₋VL _{anti-VEGF-A}- G₄S-VH _{anti-ANG-2}-(G₄S)₃-VL _{anti-ANG-2} | EVQLVESGGGLVQ PGGSLRLSCAASGF TISDYWIHWVRQA PGKGLEWVAGITP AGGYTYYADSVKG RFTISADTSKNTAY LQMNSLRAEDTAV YYCARFVFFLPYA MDYWGQGTLVTV SSGGGGSGGGGSG GGGSDIQMTQSPSS LSASVGDRVTITCR ASQDVSTAVAWYQ QKPGKAPKLLIYSA SFLYSGVPSRFSGS GSGTDFTLTISSLQP EDFATYYCKQGYA NPWTFGQGTKVEI KRGGGGSQVQLVE SGGGVVQPGRSLR LSCAASGFTFTNY GMHWGRQAPGKG LEWVAVISHDGNN KYYVDSVKGRFTI SRDNSKNTLYLQM NSLRAEDTAVYYC AREGIDFWSGLNW FDPWGQGTLVTVS SAGGGGSGGGGSG GGGSEIVLTQSPGT LSLSPGERATLSCR ASQSITGSYLAWY QQKPGQAPRLLIC GASSWATGIPDRFS GSGSGTDFTLTISR LEPEDFAVYYCQQ YSSSPITFGQGTRL EIKR | SEQ ID NO: 17 | gaggtgcagctggtggagagcggaggaggactggtgcagcca ggaggctccctgcggctgtcttgcgccgccagcggctttaccatct ccgactactggattcactgggtgagacaggcacctggcaaggga ctggagtgggtggcaggaatcaccccagcaggaggctacacata ctatgccgacagcgtgaagggccggttcaccatctccgccgatac ctctaagaacacagcctatctgcagatgaactccctgcgggccga ggacacagccgtgtactattgcgccagattcgtgttcttcctgccat acgccatggattattggggccagggcaccctggtgacagtgagct ccggtggaggtggctccggtggcggtggcagcggcggtggcg gctctgatattcagatgacacagtccccatctagcctgtctgccagc gtgggcgacagggtgaccatcacatgtagagcaagccaggacg tgagcaccgcagtggcatggtaccagcagaagcctggcaaggc cccaaagctgctgatctactccgcctctttcctgtattctggcgtgcc aagcaggtttagcgggtccggatctggaaccgacttcaccctgac aatctcctctctgcagcctgaggattttgccacatactattgcaagc agggctatgccaatccatggaccttcggccagggcacaaaggtg gagatcaagaggggcggagggggctctcaggtgcagctggttg aatctggtggcggagttgtgcagcctggcagaagcctgagactgt cttgtgccgccagcggcttcaccttcaccaactatggaatgcactg gggcagacaggcccctggcaaaggacttgaatgggtcgccgtg atcagccacgacggcaacaacaagtactacgtggacagcgtgaa gggcagattcaccatcagccgggacaacagcaagaacaccctgt acctgcagatgaacagcctgagagccgaggacaccgccgtgta ctattgtgccagagagggcatcgacttttggagcggcctgaattgg ttcgacccttggggacagggcaccctggttacagtttcttctgccg gtggaggtggctccggtggcggtggcagcggcggtggcggctc tgagatcgtgctgacacagagccctggcacactgtcactgtctcca ggcgaaagagccacactgagctgtagagccagccagagcatca ccggctcttacctggcttggtatcagcagaagcctggacaggccc ctagactgctgatttgtggcgcctcttcttgggccaccggcattccc gatagattttctggcagcggctccggcaccgacttcaccctgacaa tcagcagactggaacccgaggacttcgccgtgtactactgccagc agtacagcagcagccccatcacctttggccagggcacaagactg gaaatcaagcgg | SEQ ID NO: 40 |
| | CD5-sp-VH _{anti-VEGF-A}-(G₄S)₃-VL _{anti-VEGF-A}-G₄S-VH _{anti-ANG-2}-(G₄S)₃-VL _{anti-ANG-2} | MPMGSLQPLATLY LLGMLVASCLGEV QLVESGGGLVQPG GSLRLSCAASGFTI SDYWIHWVRQAP GKGLEWVAGITPA GGYTYYADSVKGR FTISADTSKNTAYL QMNSLRAEDTAVY YCARFVFFLPYAM DYWGQGTLVTVSS GGGGSGGGGSGG GGSDIQMTQSPSSL SASVGDRVTITCRA SQDVSTAVAWYQQ KPGKAPKLLIYSAS FLYSGVPSRFSGSG SGTDFTLTISSLQPE DFATYYCKQGYAN PWTFGQGTKVEIK RGGGGSQVQLVES GGGVVQPGRSLRL SCAASGFTFTNYG MHWGRQAPGKGL EWVAVISHDGNNK YYVDSVKGRFTISR DNSKNTLYLQMNS LRAEDTAVYYCAR EGIDFWSGLNWFD PWGQGTLVTVSSA GGGGSGGGGSGG | SEQ ID NO : 20 | atgccaatggggtccctacaacccctggcaacactatatctgctag gtatgctcgtggcctcctgtttaggagaggtgcagctggtggaga gcggaggaggactggtgcagccaggaggctccctgcggctgtc ttgcgccgccagcggctttaccatctccgactactggattcactgg gtgagacaggcacctggcaagggactggagtgggtggcaggaa tcaccccagcaggaggctacacatactatgccgacagcgtgaag ggccggttcaccatctccgccgatacctctaagaacacagcctatc tgcagatgaactccctgcgggccgaggacacagccgtgtactatt gcgccagattcgtgttcttcctgccatacgccatggattattggggc cagggcaccctggtgacagtgagctccggtggaggtggctccg gtggcggtggcagcggcggtggcggctctgatattcagatgaca cagtccccatctagcctgtctgccagcgtgggcgacagggtgac catcacatgtagagcaagccaggacgtgagcaccgcagtggcat ggtaccagcagaagcctggcaaggccccaaagctgctgatctac tccgcctctttcctgtattctggcgtgccaagcaggtttagcgggtc cggatctggaaccgacttcaccctgacaatctcctctctgcagcct gaggattttgccacatactattgcaagcagggctatgccaatccat ggaccttcggccagggcacaaaggtggagatcaagaggggcg gagggggctctcaggtgcagctggttgaatctggtggcggagttg tgcagcctggcagaagcctgagactgtcttgtgccgccagcggct tcaccttcaccaactatggaatgcactggggcagacaggcccctg gcaaaggacttgaatgggtcgccgtgatcagccacgacggcaac aacaagtactacgtggacagcgtgaagggcagattcaccatcag ccgggacaacagcaagaacaccctgtacctgcagatgaacagc ctgagagccgaggacaccgccgtgtactattgtgccagagaggg catcgacttttggagcggcctgaattggttcgacccttggggacag ggcaccctggttacagtttcttctgccggtggaggtggctccggtg gcggtggcagcggcggtggcggctctgagatcgtgctgacaca gagccctggcacactgtcactgtctccaggcgaaagagccacac tgagctgtagagccagccagagcatcaccggctcttacctggctt ggtatcagcagaagcctggacaggcccctagactgctgatttgtg | SEQ ID NO: 41 |
| | | GGSEIVLTQSPGTL SLSPGERATLSCRA SQSITGSYLAWYQ QKPGQAPRLLICG ASSWATGIPDRFSG SGSGTDFTLTISRLE PEDFAVYYCQQYS SSPITFGQGTRLEIK R | | gcgcctcttcttgggccaccggcattcccgatagattttctggcagc ggctccggcaccgacttcaccctgacaatcagcagactggaacc cgaggacttcgccgtgtactactgccagcagtacagcagcagcc ccatcacctttggccagggcacaagactggaaatcaagcgg | |
| - | CD5-sp signal peptide | MPMGSLQPLATLY LLGMLVASCLG | SEQ ID NO: 23 | atgccaatggggtccctacaacccctggcaacactatatctgctag gtatgctcgtggcctcctgtttagga | SEQ ID NO : 42 |

**Table 2. Sequence information of XMVA01 and XMVA04**

| Product Number | Sequence information | AA sequence | DNA sequence |
|---|---|---|---|
| | VH_{anti-VEGF-A} | EVQLVESGGGLVQPGGSL RLSCTASGFSLTDYYYMT WVRQAPGKGLEWVGFID PDDDPYYATWAKGRFTIS RDNSKNTLYLQMNSLRAE DTAVYYCAGGDHNSGWG LDIWGQGTLVTVSS | gaggtgcagctggttgaatctggcggaggactggttcagcctggcggatctctgagactg agctgtaccgccagcggcttcagcctgaccgactactactacatgacctgggtccgacag gcccctggcaaaggacttgagtgggtcggattcatcgaccccgacgacgatccttactac gccacatgggccaagggcagattcaccatcagccgggacaacagcaagaacaccctgt acctgcagatgaacagcctgagagccgaggacaccgccgtgtactattgtgccggcgga gatcacaatagcggctggggccttgatatttggggccagggaacactggtcaccgtgtct agt |
| | VL_{anti-VEGF-A} | EIVMTQSPSTLSASVGDR VIITCQASEIIHSWLAWYQ QKPGKAPKLLIYLASTLA SGVPSRFSGSGSGAEFTLT ISSLQPDDFATYYCQNVY LASTNGANFGQGTKLTVL G | gagatcgtgatgacacagagccccagcacactgtctgccagcgtgggagacagagtgat catcacatgccaggccagcgagatcatccacagctggctggcttggtatcagcagaagc ctggcaaggcccctaagctgctgatctacctggcctctacactggccagcggagtgccta gcagattttctggctctggatctggcgccgagttcaccctgaccatcagtagcctgcagcct gacgacttcgccacctactactgccagaacgtgtacctggccagcaccaacggcgccaa ttttggccagggcaccaagctgacagtgctggga |
| | VH_{anti-ANG-2} | QVQLVQSGAEVKKPGAS VKVSCKASGYTFTGYYM HWVRQAPGQGLEWMGW INPNSGGTNYAQKFQGRV TMTRDTSISTAYMELSRLR SDDTAVYYCARSPNPYYY DSSGYYYPGAFDIWGQGT MVTVSS | caggttcagctggttcagtctggcgccgaagtgaagaaacctggcgcctctgtgaaggtg tcctgcaaggccagcggctacacctttaccggctactacatgcactgggtccgacaggct ccaggacagggacttgaatggatgggctggatcaaccccaatagcggcggcaccaatta cgcccagaaattccagggcagagtgaccatgaccagagacaccagcatcagcaccgcc tacatggaactgagccggctgagatccgatgacaccgccgtgtactactgcgccagatct cccaatccttactactacgacagcagcgggtactactacccaggcgccttcgatatttggg gccagggcacaatggtcaccgtgtctagt |
| XMVA01 | VL_{anti-ANG-2} | SYVLTQPPSVSVAPGQTAR ITCGGNNIGSKSVHWYQQ KPGQAPVLVVYDDSDRPS GIPERFSGSNSGNTATLTIS RVEAGDEADYYCQVWDS SSDHWVFGGGTKLTVL | agctacgtgctgacacagcctccatccgtgtctgtggctccaggacagaccgccagaatc acatgcggcggcaacaacatcggcagcaagagcgtgcactggtatcagcagaagcctg gacaggctcctgtgctggtggtgtacgacgacagcgatagacctagcggcatccccgag agattcagcggcagcaattccggcaataccgccacactgaccatcagcagagtggaagc tggcgacgaggccgactactactgccaagtgtgggacagcagcagcgaccactgggttt tcggcggaggcacaaagctgacagtgctg |
| | VH_{anti-VEGF-A}-(G₄S)₃-VL_{anti-VEGF-A}- G₄S-VH_{anti-ANG-2}-(G₄S)₃-VL_{anti-ANG-2} | EVQLVESGGGLVQPGGSL RLSCTASGFSLTDYYYMT WVRQAPGKGLEWVGFID PDDDPYYATWAKGRFTIS RDNSKNTLYLQMNSLRAE DTAVYYCAGGDHNSGWG LDIWGQGTLVTVSSGGGG SGGGGSGGGGSEIVMTQS PSTLSASVGDRVIITCQAS EIIHSWLAWYQQKPGKAP KLLIYLASTLASGVPSRFS GSGSGAEFTLTISSLQPDD FATYYCQNVYLASTNGA NFGQGTKLTVLGGGGGS QVQLVQSGAEVKKPGAS VKVSCKASGYTFTGYYM HWVRQAPGQGLEWMGW INPNSGGTNYAQKFQGRV TMTRDTSISTAYMELSRLR SDDTAVYYCARSPNPYYY DSSGYYYPGAFDIWGQGT MVTVSSGGGGSGGGGSG GGGSSYVLTQPPSVSVAP GQTARITCGGNNIGSKSV HWYQQKPGQAPVLVVYD | gaggtgcagctggttgaatctggcggaggactggttcagcctggcggatctctgagactg agctgtaccgccagcggcttcagcctgaccgactactactacatgacctgggtccgacag gcccctggcaaaggacttgagtgggtcggattcatcgaccccgacgacgatccttactac gccacatgggccaagggcagattcaccatcagccgggacaacagcaagaacaccctgt acctgcagatgaacagcctgagagccgaggacaccgccgtgtactattgtgccggcgga gatcacaatagcggctggggccttgatatttggggccagggaacactggtcaccgtgtct agtggtggaggtggctccggtggcggtggcagcggcggtggcggctctgagatcgtga tgacacagagccccagcacactgtctgccagcgtgggagacagagtgatcatcacatgc caggccagcgagatcatccacagctggctggcttggtatcagcagaagcctggcaaggc ccctaagctgctgatctacctggcctctacactggccagcggagtgcctagcagattttctg gctctggatctggcgccgagttcaccctgaccatcagtagcctgcagcctgacgacttcgc cacctactactgccagaacgtgtacctggccagcaccaacggcgccaattttggccaggg caccaagctgacagtgctgggaggcggaggggggctctcaggttcagctggttcagtctg gcgccgaagtgaagaaacctggcgcctctgtgaaggtgtcctgcaaggccagcggctac acctttaccggctactacatgcactgggtccgacaggctccaggacagggacttgaatgg atgggctggatcaaccccaatagcggcggcaccaattacgcccagaaattccagggcag agtgaccatgaccagagacaccagcatcagcaccgcctacatggaactgagccggctg agatccgatgacaccgccgtgtactactgcgccagatctcccaatccttactactacgaca gcagcgggtactactacccaggcgccttcgatatttggggccagggcacaatggtcacc gtgtctagtggtggaggtggctccggtggcggtggcagcggcggtggcggctctagcta cgtgctgacacagcctccatccgtgtctgtggctccaggacagaccgccagaatcacatg cggcggcaacaacatcggcagcaagagcgtgcactggtatcagcagaagcctggacag gctcctgtgctggtggtgtacgacgacagcgatagacctagcggcatccccgagagattc agcggcagcaattccggcaataccgccacactgaccatcagcagagtggaagctggcg acgaggccgactactactgccaagtgtgggacagcagcagcgaccactgggttttcggc |
| | | DSDRPSGIPERFSGSNSGN TATLTISRVEAGDEADYYC QVWDSSSDHWVFGGGTK LTVL | ggaggcacaaagctgacagtgctg |
| | CD5-sp-VH_{anti-VEGF-A}-(G₄S)₃-VL_{anti-VEGF-A}- G₄S-VH_{anti-ANG-2}-(G₄S)₃-VL_{anti-ANG-2} | MPMGSLQPLATLYLLGML VASCLGEVQLVESGGGLV QPGGSLRLSCTASGFSLTD YYYMTWVRQAPGKGLE WVGFIDPDDDPYYATWA KGRFTISRDNSKNTLYLQ MNSLRAEDTAVYYCAGG DHNSGWGLDIWGQGTLV TVSSGGGGSGGGGSGGG GSEIVMTQSPSTLSASVGD RVIITCQASEIIHSWLAWY QQKPGKAPKLLIYLASTL ASGVPSRFSGSGSGAEFTL TISSLQPDDFATYYCQNV YLASTNGANFGQGTKLTV LGGGGGSQVQLVQSGAE VKKPGASVKVSCKASGY TFTGYYMHWVRQAPGQG LEWMGWINPNSGGTNYA QKFQGRVTMTRDTSISTA YMELSRLRSDDTAVYYCA RSPNPYYYDSSGYYYPGA FDIWGQGTMVTVSSGGG GSGGGGSGGGGSSYVLTQ PPSVSVAPGQTARITCGGN NIGSKSVHWYQQKPGQA PVLVVYDDSDRPSGIPERF SGSNSGNTATLTISRVEAG DEADYYCQVWDSSSDHW VFGGGTKLTVL | atgccaatggggtccctacaacccctggcaacactatatctgctaggtatgctcgtggcctc ctgtttaggagaggtgcagctggttgaatctggcggaggactggttcagcctggcggatct ctgagactgagctgtaccgccagcggcttcagcctgaccgactactactacatgacctgg gtccgacaggcccctggcaaaggacttgagtgggtcggattcatcgaccccgacgacga tccttactacgccacatgggccaagggcagattcaccatcagccgggacaacagcaaga acaccctgtacctgcagatgaacagcctgagagccgaggacaccgccgtgtactattgtg ccggcggagatcacaatagcggctggggccttgatatttggggccagggaacactggtc accgtgtctagtggtggaggtggctccggtggcggtggcagcggcggtggcggctctg agatcgtgatgacacagagccccagcacactgtctgccagcgtgggagacagagtgatc atcacatgccaggccagcgagatcatccacagctggctggcttggtatcagcagaagcct ggcaaggcccctaagctgctgatctacctggcctctacactggccagcggagtgcctagc agattttctggctctggatctggcgccgagttcaccctgaccatcagtagcctgcagcctga cgacttcgccacctactactgccagaacgtgtacctggccagcaccaacggcgccaatttt ggccagggcaccaagctgacagtgctgggaggcggagggggctctcaggttcagctg gttcagtctggcgccgaagtgaagaaacctggcgcctctgtgaaggtgtcctgcaaggcc agcggctacacctttaccggctactacatgcactgggtccgacaggctccaggacaggg acttgaatggatgggctggatcaaccccaatagcggcggcaccaattacgcccagaaatt ccagggcagagtgaccatgaccagagacaccagcatcagcaccgcctacatggaactg agccggctgagatccgatgacaccgccgtgtactactgcgccagatctcccaatccttact actacgacagcagcgggtactactacccaggcgccttcgatatttggggccagggcaca atggtcaccgtgtctagtggtggaggtggctccggtggcggtggcagcggcggtggcg gctctagctacgtgctgacacagcctccatccgtgtctgtggctccaggacagaccgcca gaatcacatgcggcggcaacaacatcggcagcaagagcgtgcactggtatcagcagaa gcctggacaggctcctgtgctggtggtgtacgacgacagcgatagacctagcggcatcc ccgagagattcagcggcagcaattccggcaataccgccacactgaccatcagcagagtg gaagctggcgacgaggccgactactactgccaagtgtgggacagcagcagcgaccact gggttttcggcggaggcacaaagctgacagtgctg |
| | VH_{anti-VEGF- A}(G6-23) | EVQLVESGGGLVQPGGSL RLSCAASGFTISDYWIHW VRQAPGKGLEWVAGITPA GGYTYYADSVKGRFTISA DTSKNTAYLQMNSLRAED TAVYYCARFVFFLPYAMD YWGQGTLVTVSS | gaggtgcagctggtggagagcggaggaggactggtgcagccaggaggctccctgcgg ctgtcttgcgccgccagcggctttaccatctccgactactggattcactgggtgagacagg cacctggcaagggactggagtgggtggcaggaatcaccccagcaggaggctacacata ctatgccgacagcgtgaagggccggttcaccatctccgccgatacctctaagaacacagc ctatctgcagatgaactccctgcgggccgaggacacagccgtgtactattgcgccagattc gtgttcttcctgccatacgccatggattattggggccagggcaccctggtgacagtgagct cc |
| | VL_{anti-VEGF-A}(G6-23) | DIQMTQSPSSLSASVGDR VTITCRASQDVSTAVAWY QQKPGKAPKLLIYSASFLY SGVPSRFSGSGSGTDFTLT ISSLQPEDFATYYCKQGYA NPWTFGQGTKVEIKR | gatattcagatgacacagtccccatctagcctgtctgccagcgtgggcgacagggtgacc atcacatgtagagcaagccaggacgtgagcaccgcagtggcatggtaccagcagaagc ctggcaaggccccaaagctgctgatctactccgcctctttcctgtattctggcgtgccaagc aggtttagcgggtccggatctggaaccgacttcaccctgacaatctcctctctgcagcctg aggattttgccacatactattgcaagcagggctatgccaatccatggaccttcggccaggg cacaaaggtggagatcaagagg |
| | VH_{anti-ANG-2} | QVQLVQSGAEVKKPGAS VKVSCKASGYTFTGYYM HWVRQAPGQGLEWMGW INPNSGGTNYAQKFQGRV TMTRDTSISTAYMELSRLR SDDTAVYYCARSPNPYYY DSSGYYYPGAFDIWGQGT MVTVSS | caggttcagctggttcagtctggcgccgaagtgaagaaacctggcgcctctgtgaaggtg tcctgcaaggccagcggctacacctttaccggctactacatgcactgggtccgacaggct ccaggacagggacttgaatggatgggctggatcaaccccaatagcggcggcaccaatta cgcccagaaattccagggcagagtgaccatgaccagagacaccagcatcagcaccgcc tacatggaactgagccggctgagatccgatgacaccgccgtgtactactgcgccagatct cccaatccttactactacgacagcagcgggtactactacccaggcgccttcgatatttggg gccagggcacaatggtcaccgtgtctagt |
| XMVA04 | VL_{anti-ANG-2} | SYVLTQPPSVSVAPGQTAR ITCGGNNIGSKSVHWYQQ KPGQAPVLVVYDDSDRPS GIPERFSGSNSGNTATLTIS RVEAGDEADYYCQVWDS SSDHWVFGGGTKLTVL | agctacgtgctgacacagcctccatccgtgtctgtggctccaggacagaccgccagaatc acatgcggcggcaacaacatcggcagcaagagcgtgcactggtatcagcagaagcctg gacaggctcctgtgctggtggtgtacgacgacagcgatagacctagcggcatccccgag agattcagcggcagcaattccggcaataccgccacactgaccatcagcagagtggaagc tggcgacgaggccgactactactgccaagtgtgggacagcagcagcgaccactgggttt tcggcggaggcacaaagctgacagtgctg |
| | VH_{anti-VEGF-A}-(G₄S)₃-VL_{anti-VEGF-A}- G₄S-VH_{anti-ANG-2}-(G₄S)₃-VL_{anti-ANG-2} | EVQLVESGGGLVQPGGSL RLSCAASGFTISDYWIHW VRQAPGKGLEWVAGITPA GGYTYYADSVKGRFTISA DTSKNTAYLQMNSLRAED TAVYYCARFVFFLPYAMD YWGQGTLVTVSSGGGGS GGGGSGGGGSDIQMTQSP SSLSASVGDRVTITCRASQ | gaggtgcagctggtggagagcggaggaggactggtgcagccaggaggctccctgcgg ctgtcttgcgccgccagcggctttaccatctccgactactggattcactgggtgagacagg cacctggcaagggactggagtgggtggcaggaatcaccccagcaggaggctacacata ctatgccgacagcgtgaagggccggttcaccatctccgccgatacctctaagaacacagc ctatctgcagatgaactccctgcgggccgaggacacagccgtgtactattgcgccagattc gtgttcttcctgccatacgccatggattattggggccagggcaccctggtgacagtgagct ccggtggaggtggctccggtggcggtggcagcggcggtggcggctctgatattcagatg acacagtccccatctagcctgtctgccagcgtgggcgacagggtgaccatcacatgtaga gcaagccaggacgtgagcaccgcagtggcatggtaccagcagaagcctggcaaggcc |
| | | DVSTAVAWYQQKPGKAP KLLIYSASFLYSGVPSRFS GSGSGTDFTLTISSLQPED FATYYCKQGYANPWTFG QGTKVEIKRGGGGSQVQL VQSGAEVKKPGASVKVS CKASGYTFTGYYMHWVR QAPGQGLEWMGWINPNS GGTNYAQKFQGRVTMTR DTSISTAYMELSRLRSDDT AVYYCARSPNPYYYDSSG YYYPGAFDIWGQGTMVT VSSGGGGSGGGGSGGGG SSYVLTQPPSVSVAPGQTA RITCGGNNIGSKSVHWYQ QKPGQAPVLVVYDDSDR PSGIPERFSGSNSGNTATLT ISRVEAGDEADYYCQVW DSSSDHWVFGGGTKLTVL | ccaaagctgctgatctactccgcctctttcctgtattctggcgtgccaagcaggtttagcgg gtccggatctggaaccgacttcaccctgacaatctcctctctgcagcctgaggattttgcca catactattgcaagcagggctatgccaatccatggaccttcggccagggcacaaaggtgg agatcaagaggggcggagggggctctcaggttcagctggttcagtctggcgccgaagtg aagaaacctggcgcctctgtgaaggtgtcctgcaaggccagcggctacacctttaccggc tactacatgcactgggtccgacaggctccaggacagggacttgaatggatgggctggatc aaccccaatagcggcggcaccaattacgcccagaaattccagggcagagtgaccatgac cagagacaccagcatcagcaccgcctacatggaactgagccggctgagatccgatgac accgccgtgtactactgcgccagatctcccaatccttactactacgacagcagcgggtact actacccaggcgcccttcgatatttggggccagggcacaatggtcaccgtgtctagtggtg gaggtggctccggtggcggtggcagcggcggtggcggctctagctacgtgctgacaca gcctccatccgtgtctgtggctccaggacagaccgccagaatcacatgcggcggcaaca acatcggcagcaagagcgtgcactggtatcagcagaagcctggacaggctcctgtgctg gtggtgtacgacgacagcgatagacctagcggcatccccgagagattcagcggcagca attccggcaataccgccacactgaccatcagcagagtggaagctggcgacgaggccga ctactactgccaagtgtgggacagcagcagcgaccactgggttttcggcggaggcacaa agctgacagtgctg |
| | CD5-sp-VH_{anti-VEGF-A}-(G₄S)₃-VL_{anti-VEGF-A}- G₄S-VH_{anti-ANG-2}-(G₄S)₃-VL_{anti-ANG-2} | MPMGSLQPLATLYLLGML VASCLGEVQLVESGGGLV QPGGSLRLSCAASGFTISD YWIHWVRQAPGKGLEW VAGITPAGGYTYYADSVK GRFTISADTSKNTAYLQM NSLRAEDTAVYYCARFVF FLPYAMDYWGQGTLVTV SSGGGGSGGGGSGGGGS DIQMTQSPSSLSASVGDR VTITCRASQDVSTAVAWY QQKPGKAPKLLIYSASFLY SGVPSRFSGSGSGTDFTLT ISSLQPEDFATYYCKQGYA NPWTFGQGTKVEIKRGG GGSQVQLVQSGAEVKKP GASVKVSCKASGYTFTGY YMHWVRQAPGQGLEWM GWINPNSGGTNYAQKFQ GRVTMTRDTSISTAYMEL SRLRSDDTAVYYCARSPN PYYYDSSGYYYPGAFDIW GQGTMVTVSSGGGGSGG GGSGGGGSSYVLTQPPSV SVAPGQTARITCGGNNIGS KSVHWYQQKPGQAPVLV VYDDSDRPSGIPERFSGSN SGNTATLTISRVEAGDEAD YYCQVWDSSSDHWVFGG GTKLTVL | atgccaatggggtccctacaacccctggcaacactatatctgctaggtatgctcgtggcctc ctgtttaggagaggtgcagctggtggagagcggaggaggactggtgcagccaggagg ctccctgcggctgtcttgcgccgccagcggctttaccatctccgactactggattcactggg tgagacaggcacctggcaagggactggagtgggtggcaggaatcaccccagcaggag gctacacatactatgccgacagcgtgaagggccggttcaccatctccgccgatacctctaa gaacacagcctatctgcagatgaactccctgcgggccgaggacacagccgtgtactattg cgccagattcgtgttcttcctgccatacgccatggattattggggccagggcaccctggtg acagtgagctccggtggaggtggctccggtggcggtggcagcggcggtggcggctctg atattcagatgacacagtccccatctagcctgtctgccagcgtgggcgacagggtgaccat cacatgtagagcaagccaggacgtgagcaccgcagtggcatggtaccagcagaagcct ggcaaggccccaaagctgctgatctactccgcctctttcctgtattctggcgtgccaagca ggtttagcgggtccggatctggaaccgacttcaccctgacaatctcctctctgcagcctga ggattttgccacatactattgcaagcagggctatgccaatccatggaccttcggccagggc acaaaggtggagatcaagaggggcggaggggggctctcaggttcagctggttcagtctgg cgccgaagtgaagaaacctggcgcctctgtgaaggtgtcctgcaaggccagcggctaca cctttaccggctactacatgcactgggtccgacaggctccaggacagggacttgaatgga tgggctggatcaaccccaatagcggcggcaccaattacgcccagaaattccagggcaga gtgaccatgaccagagacaccagcatcagcaccgcctacatggaactgagccggctga gatccgatgacaccgccgtgtactactgcgccagatctcccaatccttactactacgacag cagcgggtactactacccaggcgccttcgatatttggggccagggcacaatggtcaccgt gtctagtggtggaggtggctccggtggcggtggcagcggcggtggcggctctagctac gtgctgacacagcctccatccgtgtctgtggctccaggacagaccgccagaatcacatgc ggcggcaacaacatcggcagcaagagcgtgcactggtatcagcagaagcctggacag gctcctgtgctggtggtgtacgacgacagcgatagacctagcggcatccccgagagattc agcggcagcaattccggcaataccgccacactgaccatcagcagagtggaagctggcg acgaggccgactactactgccaagtgtgggacagcagcagcgaccactgggttttcggc ggaggcacaaagctgacagtgctg |

### Example 2. Measurement of expression of XMVA01, XMVA04, and XMVA09 in cells

The HEK293T cells were transfected with pAAV9-XMVA01, pAAV9-XMVA04 and pAAV9-XMVA09 plasmids respectively, the supernatant was collected, and the expression of proteins in the supernatant was detected by ELISA. The specific operation is as follows: 293T cells were resuspended in complete culture medium of DMEM containing 10% fetal bovine serum, inoculated into a culture dish, and cultured at 37°C in 5% CO₂ incubator until the confluence of the cells reaches 70%-90%, then the plasmid was transfected using Lipofectamine 2000 (Invitrogen) transfection reagent according to the instructions thereof. The culture was continued for 72 hours, and then the supernatant was collected and stored at -80°C for later use.

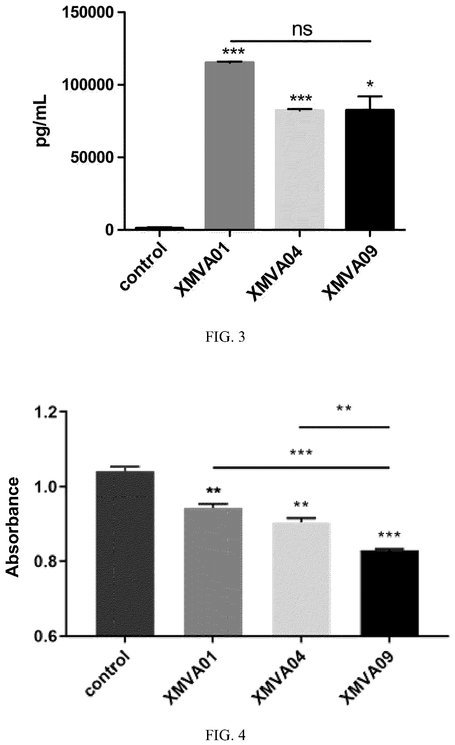

The specific detection method of ELISA was as follows: an ELISA plate (Thermo) was coated with 0.1 µg/mL VEGF165 (proteintech) protein, 100 µL per well, incubated overnight at 4°C, and the ELISA plate was washed three times with PBS containing 0.05% Tween (Sangon) for 3 minutes each time. The plates were blocked with PBS 200 µL/well containing 2% BSA (MikeBio) for 1 hour at room temperature and washed three times again. Conbercept (10 mg/mL) standard and the cell supernatants collected after transfecting HEK293T cells with pAAV9-XMVA01, pAAV9-XMVA04, and pAAV9-XMVA09 plasmids were added to the ELISA plate. The standard was diluted at 8 continuous dilutions starting at 312.5 ng/mL from the first well, and the supernatants to be tested were diluted at 1:10 or 1:50, the data were averaged, and a cell supernatant without plasmid transfection was used as a Control group, and the plate was incubated at 37°C for 1 hour, and washed three times. A 1:5000 dilutions of goat anti-human IgG antibody (Jackson Immuno Research) labeled with horseradish peroxidase was added to each well, and the plate was incubated at 37°C for 1 hour, and then washed three times. 100 µL/well of TMB chromogen solution (Beyotime) was added to each well, and reacted for 6 minutes at room temperature in the dark, and 100 µL/well of stop solution (Beyotime) was added to the above wells to terminate the reaction. A microplate reader was used to detect the OD value at 450 nm, and the contents of the sample to be tested were calculateed. The results are shown in FIG. 3. Cells transfected with pAAV9-XMVA01, pAAV9-XMVA04 and pAAV9-XMVA09 vectors can effectively express VEGF165 neutralizing protein, with significant differences compared with the Control group.

### Example 3. Effects of expressing XMVA01, XMVA04, and XMVA09 in cells on the proliferation of human retinal microvascular endothelial cells (HRMEC)

The cell density of HRMECs was adjusted to 4 × 10⁴/mL with complete culture medium (Science Cell) of the ECM containing 1% ECGS, and the cells were inoculated 100 µL/well into a flat-bottom 96-well plate; after the pAAV9-XMVA01, pAAV9-XMVA04 and pAAV9-XMVA09 plasmids were transfected into HEK293T cells, respectively, the collected cell supernatants were added into the wells at 100 µL/well, and the cell supernatant without transfected plasmids was used as a Control group, and the cells were cultured for 72 hours at 37°C in a 5% CO₂ incubator. 20 µL/well of CCK-8 solution was added to the experimental group and control group respectively according to the instructions of Cell Counting Kit-8 (CCK-8, Biosharp). After incubation for 4 hours, a microplate reader was used to detect the OD value at 450nm wavelength. The results are shown in FIG. 4. The protein expressed by pAAV9-XMVA09 can effectively inhibit the proliferation of HRMECs under the stimulation of ECGS, and the inhibition effect is significantly better than that of pAAV9-XMVA01 and pAAV9-XMVA04.

### Example 4. Effects of expressing XMVA01, XMVA04, and XMVA09 in cells on the tube formation of HRMECs

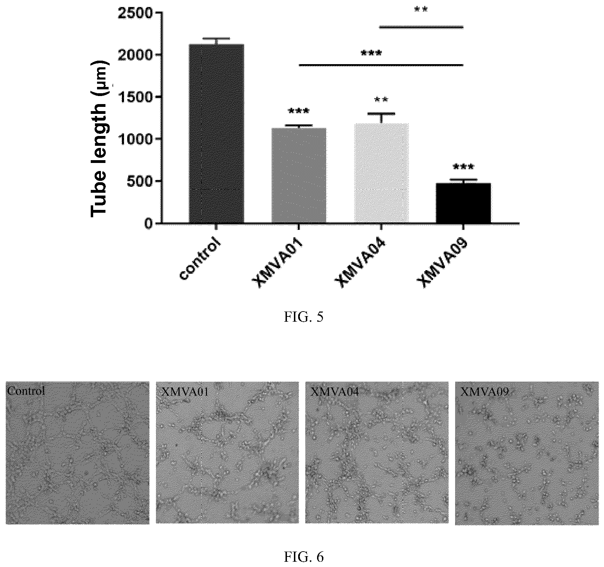

The thawed matrigel (Corning) was uniformly spreaded in a flat-bottom 96-well plate, 50 µL/well, and then the plate was incubated at 37°C for 1 hour in a 5% CO₂ incubator; after the pAAV9-XMVA01, pAAV9-XMVA04 and pAAV9-XMVA09 plasmids were transfected into HEK293T cells, respectively, the collected cell supernatants were used to treat HRMECs, the cell supernatant without transfected plasmids was used as a control group, and the cells were resuspended with a basal medium of ECM without growth factors and serum after cultured for 48 hours, adjusted the cell density to 2 × 10⁵/mL, and inoculated at 100 µL/well in a 96-well plate containing matrigel, and cultured in a 5% CO₂ incubator at 37°C, and observed once every 2 hours; after 4 to 8 hours, the cells were observed and photographed under a microscope, and the influence of the supernatants to be tested on the formation of HRMECs tubes were recorded. The results are shown in FIG. 5. The protein expressed by pAAV9-XMVA09 can effectively inhibit the formation of HRMECs tubes, and the inhibition effect is significantly better than that of pAAV9-XMVA01 and pAAV9-XMVA04.

### Example 5. Construction of other plasmid vectors

### Scheme 1

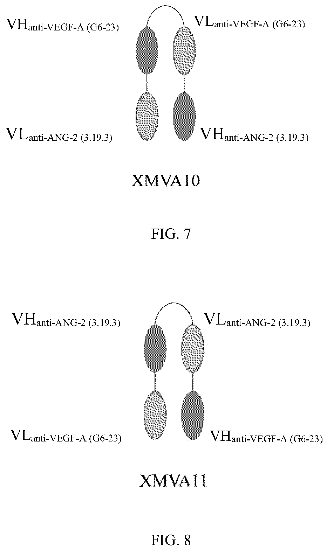

The VH amino acid sequence and the VL amino acid sequence of anti-VEGF, the VH amino acid sequence and the VL amino acid sequence of anti-ANG-2 were linked with G₄S, (G₄S)₃ and G₄S peptide linkers, respectively, with a secretory signal peptide CD5-sp nucleotide sequence added to the N-terminus to form an open reading frame with the structure of CD5-sp - VL_{anti-ANG-2} - G₄S - VH_{anti-VEGF-A} - (G₄S)₃ - VL_{anti-VEGF-A} - GaS - VH_{anti-ANG-2}; the nucleotide sequence was designed according to human codon preference, with a BamH I cleavage site introduced at the 5' terminus, and an EcoR V cleavage site introduced at the 3' terminus, and was named as XMVA10 ( FIG. 7). The VH amino acid sequence and the VL amino acid sequence of anti-VEGF, the VH amino acid sequence and the VL amino acid sequence of anti-ANG-2 were identical to XMVA09.

### Scheme 2

The VH amino acid sequence and the VL amino acid sequence of anti-VEGF, the VH amino acid sequence and the VL amino acid sequence of anti-ANG-2 were linked with G₄S, (G₄S)₃ and G₄S peptide linkers, respectively, with a secretory signal peptide CD5-sp nucleotide sequence added to the N-terminus to form an open reading frame with the structure of CD5-sp - VL_{anti-VEGF-A} - G₄S - VH_{anti-ANG-2} - (G₄S)₃ - VL_{anti-ANG-2} - G₄S - VH_{anti-VEGF-A}; the nucleotide sequence was designed according to human codon preference, with a the BamH I cleavage site introduced at the 5' terminus, and an EcoR V cleavage site introduced at the 3' terminus, and was named as XMVA11 ( FIG. 8). The VH amino acid sequence and the VL amino acid sequence of anti-VEGF, the VH amino acid sequence and the VL amino acid sequence of anti-ANG-2 were identical to XMVA09.

### Scheme 3

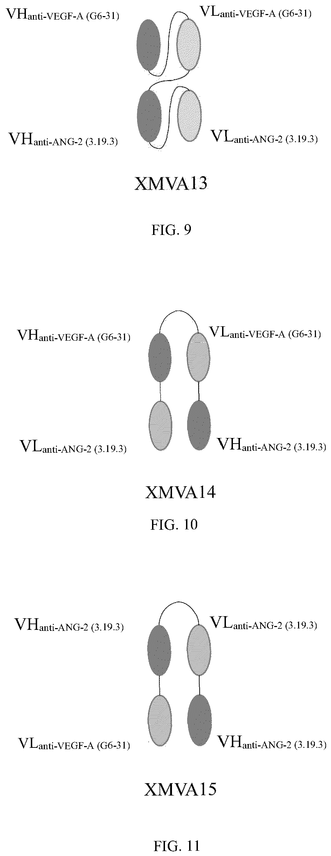

The VH amino acid sequence and the VL amino acid sequence of anti-VEGF, the VH amino acid sequence and the VL amino acid sequence of anti-ANG-2 were linked with (G₄S)₃, G₄S and (G₄S)₃ peptide linkers, respectively, with a secretory signal peptide CD5-sp nucleotide sequence added to the N-terminus to form an open reading frame with the structure CD5-sp-VH_{anti-VEGF-A} - (G₄S)₃ - VL_{anti-VEGF-A} - GaS - VH_{anti-ANG-2}- (G₄S)₃ - VL_{anti-ANG-2}; the nucleotide sequence was designed according to human codon preference, with a BamH I cleavage site introduced at the 5' terminus, and an EcoR V cleavage site introduced at the 3' terminus, and was named as XMVA13 (FIG. 9).

### Scheme 4

The VH amino acid sequence and the VL amino acid sequence of anti-VEGF, the VH amino acid sequence and the VL amino acid sequence of anti-ANG-2 were linked with G₄S, (G₄S)₃ and G₄S peptide linkers, respectively, with a secretory signal peptide CD5-sp nucleotide sequence added to the N-terminus to form an open reading frame with the structure of CD5-sp - VL _{anti-ANG-2} - G₄S - VH _{anti-VEGF-A} - (G₄S)₃ - VL _{anti-VEGF-A} - GaS - VH _{anti-ANG-2}; the nucleotide sequence was designed according to human codon preference, with a BamH I cleavage site introduced at the 5' terminus, and an EcoR V cleavage site introduced at the 3' terminus, and was named as XMVA14 ( FIG. 10). The VH amino acid sequence and the VL amino acid sequence of anti-VEGF, the VH amino acid sequence and the VL amino acid sequence of anti-ANG-2 were identical to XMVA13.

### Scheme 5

The VH amino acid sequence and the VL amino acid sequence of anti-VEGF, the VH amino acid sequence and the VL amino acid sequence of anti-ANG-2 were linked with G₄S, (G₄S)₃ and G₄S peptide linkers, respectively, with a secretory signal peptide CD5-sp nucleotide sequence added to the N-terminus to form an open reading frame with the structure of CD5-sp-VL_{anti-VEGF-A} - GaS - VH_{anti-ANG-2} - (G₄S)₃-VL_{anti-ANG-2} - G₄S - VH_{anti-VEGF-A}; the nucleotide sequence was designed according to human codon preference, with a BamH I cleavage site introduced at the 5' terminus, and an EcoR V cleavage site introduced at the 3' terminus, and was named as XMVA15 ( FIG. 11). The VH amino acid sequence and the VL amino acid sequence of anti-VEGF, the VH amino acid sequence and the VL amino acid sequence of anti-ANG-2 were identical to XMVA13.

XMVA10, XMVA11, XMVA13, XMVA14 and XMVA15 vectors were constructed through conventional molecular biology operations such as ligation, transformation, cloning screening and identification, and high-quality plasmid DNA was obtained for later use by using an endotoxin-free plasmid extraction kit (MN).

According to the method described in Example 2, plasmids were transfected into HEK293T cells, respectively, supernatants were collected, and expression of protein in supernatant was detected by ELISA. The effect of expressing the above vectors in cells on the proliferation of HRMECs was detected according to the method described in Example 3. The effect of expressing the above plasmids in cells on HRMECs tube formation was detected according to the method described in Example 4. Recombinant AAV viruses were prepared and identified according to the method described in Example 5.

**Table 3. Sequence information of XMVA10-XMVA15**

| Product Number | Sequence information | AA sequence | | DNA sequence | |
|---|---|---|---|---|---|
| XMVA10 | VL_{anti-ANG-2}-G₄S-VH_{anti-VEGF-A}-(G₄S)3-VL_{anti-VEGF-A}-G₄S-VH_{anti-ANG-2} | EIVLTQSPGTLSLSPGERAT LSCRASQSITGSYLAWYQ QKPGQAPRLLICGASSWA TGIPDRFSGSGSGTDFTLTI SRLEPEDFAVYYCQQYSSS PITFGQGTRLEIKRGGGGS EVQLVESGGGLVQPGGSL RLSCAASGFTISDYWIHW VRQAPGKGLEWVAGITPA GGYTYYADSVKGRFTISA DTSKNTAYLQMNSLRAED TAVYYCARFVFFLPYAMD YWGQGTLVTVSSGGGGS GGGGSGGGGSDIQMTQSP SSLSASVGDRVTITCRASQ DVSTAVAWYQQKPGKAP KLLIYSASFLYSGVPSRFS GSGSGTDFTLTISSLQPEDF ATYYCKQGYANPWTFGQ GTKVEIKRGGGGSQVQLV ESGGGVVQPGRSLRLSCA ASGFTFTNYGMHWGRQA PGKGLEWVAVISHDGNNK YYVDSVKGRFTISRDNSK NTLYLQMNSLRAEDTAVY YCAREGIDFWSGLNWFDP WGQGTLVTVSSA | SEQ ID NO: 18 | gagatcgtgctgacacagagccctggcacactgtcactgtctccag gcgaaagagccacactgagctgtagagccagccagagcatcaccg gctcttacctggcttggtatcagcagaagcctggacaggcccctaga ctgctgatttgtggcgcctcttcttgggccaccggcattcccgatagat tttctggcagcggctccggcaccgacttcaccctgacaatcagcaga ctggaacccgaggacttcgccgtgtactactgccagcagtacagca gcagccccatcacctttggccagggcacaagactggaaatcaagc ggggcggagggggctctgaggtgcagctggtggagagcggagg aggactggtgcagccaggaggctccctgcggctgtcttgcgccgcc agcggctttaccatctccgactactggattcactgggtgagacaggc acctggcaagggactggagtgggtggcaggaatcaccccagcag gaggctacacatactatgccgacagcgtgaagggccggttcaccat ctccgccgatacctctaagaacacagcctatctgcagatgaactccct gcgggccgaggacacagccgtgtactattgcgccagattcgtgttct tcctgccatacgccatggattattggggccagggcaccctggtgaca gtgagctccggtggaggtggctccggtggcggtggcagcggcggt ggcggctctgatattcagatgacacagtccccatctagcctgtctgcc agcgtgggcgacagggtgaccatcacatgtagagcaagccaggac gtgagcaccgcagtggcatggtaccagcagaagcctggcaaggcc ccaaagctgctgatctactccgcctctttcctgtattctggcgtgccaa gcaggtttagcgggtccggatctggaaccgacttcaccctgacaatc tcctctctgcagcctgaggattttgccacatactattgcaagcagggct atgccaatccatggaccttcggccagggcacaaaggtggagatcaa gaggggcggagggggctctcaggtgcagctggttgaatctggtgg cggagttgtgcagcctggcagaagcctgagactgtcttgtgccgcc agcggcttcaccttcaccaactatggaatgcactggggcagacagg cccctggcaaaggacttgaatgggtcgccgtgatcagccacgacg gcaacaacaagtactacgtggacagcgtgaagggcagattcaccat cagccgggacaacagcaagaacaccctgtacctgcagatgaacag | SEQ ID NO: 43 |
| | | | | cctgagagccgaggacaccgccgtgtactattgtgccagagaggg catcgacttttggagcggcctgaattggttcgacccttggggacagg gcaccctggttacagtttcttctgcc | |
| | CD5-sp-VL_{anti-ANG-2}-G₄S-VH_{anti-VEGF-A}-(G₄S)₃-VL_{anti-VEGF-A}-G₄S-VH_{anti-ANG-2} | MPMGSLQPLATLYLLGML VASCLGEIVLTQSPGTLSLS PGERATLSCRASQSITGSY LAWYQQKPGQAPRLLICG ASSWATGIPDRFSGSGSGT DFTLTISRLEPEDFAVYYC QQYSSSPITFGQGTRLEIK RGGGGSEVQLVESGGGLV QPGGSLRLSCAASGFTISD YWIHWVRQAPGKGLEWV AGITPAGGYTYYADSVKG RFTISADTSKNTAYLQMNS LRAEDTAVYYCARFVFFL PYAMDYWGQGTLVTVSS GGGGSGGGGSGGGGSDIQ MTQSPSSLSASVGDRVTIT CRASQDVSTAVAWYQQKP GKAPKLLIYSASFLYSGVP SRFSGSGSGTDFTLTISSLQ PEDFATYYCKQGYANPWT FGQGTKVEIKRGGGGSQV QLVESGGGVVQPGRSLRL SCAASGFTFTNYGMHWG RQAPGKGLEWVAVISHDG NNKYYVDSVKGRFTISRD NSKNTLYLQMNSLRAEDT AVYYCAREGIDFWSGLN WFDPWGQGTLVTVSSA | SEQ ID NO: 21 | atgccaatggggtccctacaacccctggcaacactatatctgctaggt atgctcgtggcctcctgtttaggagagatcgtgctgacacagagccc tggcacactgtcactgtctccaggcgaaagagccacactgagctgta gagccagccagagcatcaccggctcttacctggcttggtatcagca gaagcctggacaggcccctagactgctgatttgtggcgcctcttcttg ggccaccggcattcccgatagattttctggcagcggctccggcacc gacttcaccctgacaatcagcagactggaacccgaggacttcgccg tgtactactgccagcagtacagcagcagccccatcacctttggccag ggcacaagactggaaatcaagcggggcggagggggctctgaggt gcagctggtggagagcggaggaggactggtgcagccaggaggct ccctgcggctgtcttgcgccgccagcggctttaccatctccgactact ggattcactgggtgagacaggcacctggcaagggactggagtggg tggcaggaatcaccccagcaggaggctacacatactatgccgaca gcgtgaagggccggttcaccatctccgccgatacctctaagaacac agcctatctgcagatgaactccctgcgggccgaggacacagccgtg tactattgcgccagattcgtgttcttcctgccatacgccatggattattg gggccagggcaccctggtgacagtgagctccggtggaggtggctc cggtggcggtggcagcggcggtggcggctctgatattcagatgaca cagtccccatctagcctgtctgccagcgtgggcgacagggtgacca tcacatgtagagcaagccaggacgtgagcaccgcagtggcatggt accagcagaagcctggcaaggccccaaagctgctgatctactccgc ctctttcctgtattctggcgtgccaagcaggtttagcgggtccggatct ggaaccgacttcaccctgacaatctcctctctgcagcctgaggatttt gccacatactattgcaagcagggctatgccaatccatggaccttcgg ccagggcacaaaggtggagatcaagaggggcggagggggctctc aggtgcagctggttgaatctggtggcggagttgtgcagcctggcag aagcctgagactgtcttgtgccgccagcggcttcaccttcaccaacta tggaatgcactggggcagacaggcccctggcaaaggacttgaatg ggtcgccgtgatcagccacgacggcaacaacaagtactacgtgga cagcgtgaagggcagattcaccatcagccgggacaacagcaagaa caccctgtacctgcagatgaacagcctgagagccgaggacaccgc cgtgtactattgtgccagagagggcatcgacttttggagcggcctga attggttcgacccttggggacagggcaccctggttacagtttcttctgc c | SEQ ID NO: 44 |
| XMVA11 | VL_{anti-VEGF-A}-G₄S - VH_{anti-ANG-2}-(G₄S)₃-VL_{anti-ANG-2}-G₄S -VH_{anti-VEGF-A} | DIQMTQSPSSLSASVGDRV TITCRASQDVSTAVAWYQ QKPGKAPKLLIYSASFLYS GVPSRFSGSGSGTDFTLTIS SLQPEDFATYYCKQGYAN PWTFGQGTKVEIKRGGGG SQVQLVESGGGVVQPGRS LRLSCAASGFTFTNYGMH WGRQAPGKGLEWVAVISH DGNNKYYVDSVKGRFTIS RDNSKNTLYLQMNSLRAE DTAVYYCAREGIDFWSGL NWFDPWGQGTLVTVSSA GGGGSGGGGSGGGGSEIV LTQSPGTLSLSPGERATLS CRASQSITGSYLAWYQQK PGQAPRLLICGASSWATGI PDRFSGSGSGTDFTLTISRL EPEDFAVYYCQQYSSSPIT FGQGTRLEIKRGGGGSEV QLVESGGGLVQPGGSLRL SCAASGFTISDYWIHWVR QAPGKGLEWVAGITPAGG YTYYADSVKGRFTISADTS KNTAYLQMNSLRAEDTAV YYCARFVFFLPYAMDYW GQGTLVTVSS | SEQ ID NO: 19 | gatattcagatgacacagtccccatctagcctgtctgccagcgtggg cgacagggtgaccatcacatgtagagcaagccaggacgtgagcac cgcagtggcatggtaccagcagaagcctggcaaggccccaaagct gctgatctactccgcctctttcctgtattctggcgtgccaagcaggttta gcgggtccggatctggaaccgacttcaccctgacaatctcctctctg cagcctgaggattttgccacatactattgcaagcagggctatgccaat ccatggaccttcggccagggcacaaaggtggagatcaagagggg cggagggggctctcaggtgcagctggttgaatctggtggcggagtt gtgcagcctggcagaagcctgagactgtcttgtgccgccagcggct tcaccttcaccaactatggaatgcactggggcagacaggcccctgg caaaggacttgaatgggtcgccgtgatcagccacgacggcaacaa caagtactacgtggacagcgtgaagggcagattcaccatcagccgg gacaacagcaagaacaccctgtacctgcagatgaacagcctgaga gccgaggacaccgccgtgtactattgtgccagagagggcatcgact tttggagcggcctgaattggttcgacccttggggacagggcaccctg gttacagtttcttctgccggtggaggtggctccggtggcggtggcag cggcggtggcggctctgagatcgtgctgacacagagccctggcac actgtcactgtctccaggcgaaagagccacactgagctgtagagcc agccagagcatcaccggctcttacctggcttggtatcagcagaagcc tggacaggcccctagactgctgatttgtggcgcctcttcttgggccac cggcattcccgatagattttctggcagcggctccggcaccgacttca ccctgacaatcagcagactggaacccgaggacttcgccgtgtacta ctgccagcagtacagcagcagccccatcacctttggccagggcaca agactggaaatcaagcggggcggagggggctctgaggtgcagct ggtggagagcggaggaggactggtgcagccaggaggctccctgc ggctgtcttgcgccgccagcggctttaccatctccgactactggattc actgggtgagacaggcacctggcaagggactggagtgggtggca ggaatcaccccagcaggaggctacacatactatgccgacagcgtg aagggccggttcaccatctccgccgatacctctaagaacacagccta tctgcagatgaactccctgcgggccgaggacacagccgtgtactatt gcgccagattcgtgttcttcctgccatacgccatggattattggggcc agggcaccctggtgacagtgagctcc | SEQ ID NO: 45 |
| | CD5-sp-VL | MPMGSLQPLATLYLLGML | SEQ | atgccaatggggtccctacaacccctggcaacactatatctgctaggt | SEQ |
| | anti-VEGF-A-G₄S -VH_{anti-ANG-2}-(G₄S)₃-VL_{anti-ANG-2}-G₄S -VH_{anti-VEGF-A} | VASCLGDIQMTQSPSSLSA SVGDRVTITCRASQDVSTA VAWYQQKPGKAPKLLIYS ASFLYSGVPSRFSGSGSGT DFTLTISSLQPEDFATYYC KQGYANPWTFGQGTKVEI KRGGGGSQVQLVESGGG VVQPGRSLRLSCAASGFT FTNYGMHWGRQAPGKGL EWVAVISHDGNNKYYVDS VKGRFTISRDNSKNTLYLQ MNSLRAEDTAVYYCAREG IDFWSGLNWFDPWGQGT LVTVSSAGGGGSGGGGSG GGGSEIVLTQSPGTLSLSP GERATLSCRASQSITGSYL AWYQQKPGQAPRLLICGA SSWATGIPDRFSGSGSGTD FTLTISRLEPEDFAVYYCQ QYSSSPITFGQGTRLEIKR GGGGSEVQLVESGGGLVQ PGGSLRLSCAASGFTISDY WIHWVRQAPGKGLEWVA GITPAGGYTYYADSVKGR FTISADTSKNTAYLQMNSL RAEDTAVYYCARFVFFLP YAMDYWGQGTLVTVSS | ID NO: 22 | atgctcgtggcctcctgtttaggagatattcagatgacacagtccccat ctagcctgtctgccagcgtgggcgacagggtgaccatcacatgtag agcaagccaggacgtgagcaccgcagtggcatggtaccagcaga agcctggcaaggccccaaagctgctgatctactccgcctctttcctgt attctggcgtgccaagcaggtttagcgggtccggatctggaaccga cttcaccctgacaatctcctctctgcagcctgaggattttgccacatac tattgcaagcagggctatgccaatccatggaccttcggccagggca caaaggtggagatcaagaggggcggagggggctctcaggtgcag ctggttgaatctggtggcggagttgtgcagcctggcagaagcctga gactgtcttgtgccgccagcggcttcaccttcaccaactatggaatgc actggggcagacaggcccctggcaaaggacttgaatgggtcgccg tgatcagccacgacggcaacaacaagtactacgtggacagcgtga agggcagattcaccatcagccgggacaacagcaagaacaccctgt acctgcagatgaacagcctgagagccgaggacaccgccgtgtact attgtgccagagagggcatcgacttttggagcggcctgaattggttc gacccttggggacagggcaccctggttacagtttcttctgccggtgg aggtggctccggtggcggtggcagcggcggtggcggctctgagat cgtgctgacacagagccctggcacactgtcactgtctccaggcgaa agagccacactgagctgtagagccagccagagcatcaccggctctt acctggcttggtatcagcagaagcctggacaggcccctagactgct gatttgtggcgcctcttcttgggccaccggcattcccgatagattttct ggcagcggctccggcaccgacttcaccctgacaatcagcagactg gaacccgaggacttcgccgtgtactactgccagcagtacagcagca gccccatcacctttggccagggcacaagactggaaatcaagcggg gcggagggggctctgaggtgcagctggtggagagcggaggagg actggtgcagccaggaggctccctgcggctgtcttgcgccgccagc ggctttaccatctccgactactggattcactgggtgagacaggcacct ggcaagggactggagtgggtggcaggaatcaccccagcaggagg ctacacatactatgccgacagcgtgaagggccggttcaccatctccg ccgatacctctaagaacacagcctatctgcagatgaactccctgcgg gccgaggacacagccgtgtactattgcgccagattcgtgttcttcctg ccatacgccatggattattggggccagggcaccctggtgacagtga gctcc | ID NO: 46 |
| | VH_{anti-VEGF-A}(G6-31)-CDR1 | DYWIH | SEQ ID NO: 1 | gactactggattcac | SEQ ID NO: 24 |
| | VH_{anti-VEGF-A}(G6-31)-CDR2 | GITPAGGYTYYADSVKG | SEQ ID NO: 2 | ggaatcaccccagcaggaggctacacatactatgccgacagcgtg aagggc | SEQ ID NO: 25 |
| | VH_{anti-VEGF-A}(G6-31)-CDR3 | FVFFLPYAMDY | SEQ ID NO: 3 | ttcgtgttcttcctgccatacgccatggattat | SEQ ID NO: 26 |
| XMVA13 | VH_{anti-VEGF-A}(G6-31) | EVQLVESGGGLVQPGGSL RLSCAASGFTISDYWIHW VRQAPGKGLEWVAGITPA GGYTYYADSVKGRFTISA DTSKNTAYLQMNSLRAED TAVYYCARFVFFLPYAMD YWGQGTLVTVSS | SEQ ID NO: 13 | gaggtgcagctggtggagagcggaggaggactggtgcagccagg aggctccctgcggctgtcttgcgccgccagcggctttaccatctccg actactggattcactgggtgagacaggcacctggcaagggactgga gtgggtggcaggaatcaccccagcaggaggctacacatactatgcc gacagcgtgaagggccggttcaccatctccgccgatacctctaaga acacagcctatctgcagatgaactccctgcgggccgaggacacag ccgtgtactattgcgccagattcgtgttcttcctgccatacgccatgga ttattggggccagggcaccctggtgacagtgagctcc | SEQ ID NO: 27 |
| | VL_{anti-VEGF-A}(G6-31)-CDR1 | RASQDVSTAVA | SEQ ID NO: 4 | agagcaagccaggacgtgagcaccgcagtggca | SEQ ID NO: 28 |
| | VL_{anti-VEGF-A}(G6-31)-CDR2 | SASFLYS | SEQ ID NO: 5 | tccgcctctttcctgtattct | SEQ ID NO: 29 |
| | VL_{anti-VEGF-A}(G6-31)-CDR3 | QQGYGNPFT | SEQ ID NO: 47 | cagcagggctatggcaatccattcacc | SEQ ID NO: 55 |
| | VL_{anti-VEGF-A}(G6-31) | DIQMTQSPSSLSASVGDRV TITCRASQDVSTAVAWYQ QKPGKAPKLLIYSASFLYS GVPSRFSGSGSGTDFTLTIS | SEQ ID NO: 48 | gatattcagatgacacagtccccatctagcctgtctgccagcgtggg cgacagggtgaccatcacatgtagagcaagccaggacgtgagcac cgcagtggcatggtaccagcagaagcctggcaaggccccaaagct gctgatctactccgcctctttcctgtattctggcgtgccaagcaggttta | SEQ ID NO: 56 |
| | | SLQPEDFATYYCQQGYGN PFTFGQGTKVEIKR | | gcgggtccggatctggaaccgacttcaccctgacaatctcctctctg cagcctgaggattttgccacatactattgccagcagggctatggcaat ccattcacctttggccagggcacaaaggtggagatcaagagg | |
| | VH_{anti-ANG-2}(3.19.3) | QVQLVESGGGVVQPGRSL RLSCAASGFTFTNYGMH WGRQAPGKGLEWVAVISH DGNNKYYVDSVKGRFTIS RDNSKNTLYLQMNSLRAE DTAVYYCAREGIDFWSGL NWFDPWGQGTLVTVSSA | SEQ ID NO :15 | caggtgcagctggttgaatctggtggcggagttgtgcagcctggca gaagcctgagactgtcttgtgccgccagcggcttcaccttcaccaac tatggaatgcactggggcagacaggcccctggcaaaggacttgaat gggtcgccgtgatcagccacgacggcaacaacaagtactacgtgg acagcgtgaagggcagattcaccatcagccgggacaacagcaaga acaccctgtacctgcagatgaacagcctgagagccgaggacaccg ccgtgtactattgtgccagagagggcatcgacttttggagcggcctg aattggttcgacccttggggacagggcaccctggttacagtttcttctg cc | SEQ ID NO: 35 |
| | VL_{anti-ANG-2}(3.19.3) | EIVLTQSPGTLSLSPGERAT LSCRASQSITGSYLAWYQ QKPGQAPRLLICGASSWA TGIPDRFSGSGSGTDFTLTI SRLEPEDFAVYYCQQYSSS PITFGQGTRLEIKR | SEQ ID NO: 16 | gagatcgtgctgacacagagccctggcacactgtcactgtctccag gcgaaagagccacactgagctgtagagccagccagagcatcaccg gctcttacctggcttggtatcagcagaagcctggacaggcccctaga ctgctgatttgtggcgcctcttcttgggccaccggcattcccgatagat tttctggcagcggctccggcaccgacttcaccctgacaatcagcaga ctggaacccgaggacttcgccgtgtactactgccagcagtacagca gcagccccatcacctttggccagggcacaagactggaaatcaagc gg | SEQ ID NO: 39 |
| | VH_{anti-VEGF-A}- (G₄S)₃-VL_{anti-VEGF-A}- G₄S-VH_{anti-ANG-2}- (G₄S)₃-VL_{anti-ANG-2} | EVQLVESGGGLVQPGGSL RLSCAASGFTISDYWIHW VRQAPGKGLEWVAGITPA GGYTYYADSVKGRFTISA DTSKNTAYLQMNSLRAED TAVYYCARFVFFLPYAMD YWGQGTLVTVSSGGGGS GGGGSGGGGSDIQMTQSP SSLSASVGDRVTITCRASQ DVSTAVAWYQQKPGKAP KLLIYSASFLYSGVPSRFS GSGSGTDFTLTISSLQPEDF ATYYCQQGYGNPFTFGQG TKVEIKRGGGGSQVQLVE SGGGVVQPGRSLRLSCAA SGFTFTNYGMHWGRQAP GKGLEWVAVISHDGNNKY YVDSVKGRFTISRDNSKN TLYLQMNSLRAEDTAVYY CAREGIDFWSGLNWFDP WGQGTLVTVSSAGGGGS GGGGSGGGGSEIVLTQSP GTLSLSPGERATLSCRASQ SITGSYLAWYQQKPGQAP RLLICGASSWATGIPDRFS GSGSGTDFTLTISRLEPEDF AVYYCQQYSSSPITFGQGT RLEIKR | SEQ ID NO: 49 | gaggtgcagctggtggagagcggaggaggactggtgcagccagg aggctccctgcggctgtcttgcgccgccagcggctttaccatctccg actactggattcactgggtgagacaggcacctggcaagggactgga gtgggtggcaggaatcaccccagcaggaggctacacatactatgcc gacagcgtgaagggccggttcaccatctccgccgatacctctaaga acacagcctatctgcagatgaactccctgcgggccgaggacacag ccgtgtactattgcgccagattcgtgttcttcctgccatacgccatgga ttattggggccagggcaccctggtgacagtgagctccggtggaggt ggctccggtggcggtggcagcggcggtggcggctctgatattcag atgacacagtccccatctagcctgtctgccagcgtgggcgacaggg tgaccatcacatgtagagcaagccaggacgtgagcaccgcagtgg catggtaccagcagaagcctggcaaggccccaaagctgctgatcta ctccgcctctttcctgtattctggcgtgccaagcaggtttagcgggtcc ggatctggaaccgacttcaccctgacaatctcctctctgcagcctgag gattttgccacatactattgccagcagggctatggcaatccattcacct ttggccagggcacaaaggtggagatcaagaggggcggaggggg ctctcaggtgcagctggttgaatctggtggcggagttgtgcagcctg gcagaagcctgagactgtcttgtgccgccagcggcttcaccttcacc aactatggaatgcactggggcagacaggcccctggcaaaggactt gaatgggtcgccgtgatcagccacgacggcaacaacaagtactac gtggacagcgtgaagggcagattcaccatcagccgggacaacagc aagaacaccctgtacctgcagatgaacagcctgagagccgaggac accgccgtgtactattgtgccagagagggcatcgacttttggagcgg cctgaattggttcgacccttggggacagggcaccctggttacagtttc ttctgccggtggaggtggctccggtggcggtggcagcggcggtgg cggctctgagatcgtgctgacacagagccctggcacactgtcactgt ctccaggcgaaagagccacactgagctgtagagccagccagagca tcaccggctcttacctggcttggtatcagcagaagcctggacaggcc cctagactgctgatttgtggcgcctcttcttgggccaccggcattccc gatagattttctggcagcggctccggcaccgacttcaccctgacaat cagcagactggaacccgaggacttcgccgtgtactactgccagcag tacagcagcagccccatcacctttggccagggcacaagactggaaa tcaagcgg | SEQ ID NO: 57 |
| | CD5-sp-VH_{anti-VEGF-A}- (G₄S)₃-VL_{anti-VEGF-A}- G₄S-VH_{anti-ANG-2}- (G₄S)₃-VL_{anti-ANG-2} | MPMGSLQPLATLYLLGML VASCLGEVQLVESGGGLV QPGGSLRLSCAASGFTISD YWIHWVRQAPGKGLEWV AGITPAGGYTYYADSVKG RFTISADTSKNTAYLQMNS LRAEDTAVYYCARFVFFL PYAMDYWGQGTLVTVSS GGGGSGGGGSGGGGSDIQ MTQSPSSLSASVGDRVTIT CRASQDVSTAVAWYQQKP GKAPKLLIYSASFLYSGVP SRFSGSGSGTDFTLTISSLQ PEDFATYYCQQGYGNPFT FGQGTKVEIKRGGGGSQV QLVESGGGVVQPGRSLRL SCAASGFTFTNYGMHWG | SEQ ID NO: 50 | atgccaatggggtccctacaacccctggcaacactatatctgctaggt atgctcgtggcctcctgtttaggagaggtgcagctggtggagagcg gaggaggactggtgcagccaggaggctccctgcggctgtcttgcg ccgccagcggctttaccatctccgactactggattcactgggtgaga caggcacctggcaagggactggagtgggtggcaggaatcacccc agcaggaggctacacatactatgccgacagcgtgaagggccggtt caccatctccgccgatacctctaagaacacagcctatctgcagatga actccctgcgggccgaggacacagccgtgtactattgcgccagatt cgtgttcttcctgccatacgccatggattattggggccagggcaccct ggtgacagtgagctccggtggaggtggctccggtggcggtggcag cggcggtggcggctctgatattcagatgacacagtccccatctagcc tgtctgccagcgtgggcgacagggtgaccatcacatgtagagcaag ccaggacgtgagcaccgcagtggcatggtaccagcagaagcctgg caaggccccaaagctgctgatctactccgcctctttcctgtattctggc gtgccaagcaggtttagcgggtccggatctggaaccgacttcaccct gacaatctcctctctgcagcctgaggattttgccacatactattgccag cagggctatggcaatccattcacctttggccagggcacaaaggtgg | SEQ ID NO: 58 |
| | | RQAPGKGLEWVAVISHDG NNKYYVDSVKGRFTISRD NSKNTLYLQMNSLRAEDT AVYYCAREGIDFWSGLN WFDPWGQGTLVTVSSAG GGGSGGGGSGGGGSEIVL TQSPGTLSLSPGERATLSC RASQSITGSYLAWYQQKP GQAPRLLICGASSWATGIP DRFSGSGSGTDFTLTISRLE PEDFAVYYCQQYSSSPITF GQGTRLEIKR | | agatcaagaggggcggagggggctctcaggtgcagctggttgaat ctggtggcggagttgtgcagcctggcagaagcctgagactgtcttgt gccgccagcggcttcaccttcaccaactatggaatgcactggggca gacaggcccctggcaaaggacttgaatgggtcgccgtgatcagcc acgacggcaacaacaagtactacgtggacagcgtgaagggcagat tcaccatcagccgggacaacagcaagaacaccctgtacctgcagat gaacagcctgagagccgaggacaccgccgtgtactattgtgccaga gagggcatcgacttttggagcggcctgaattggttcgacccttgggg acagggcaccctggttacagtttcttctgccggtggaggtggctccg gtggcggtggcagcggcggtggcggctctgagatcgtgctgacac agagccctggcacactgtcactgtctccaggcgaaagagccacact gagctgtagagccagccagagcatcaccggctcttacctggcttggt atcagcagaagcctggacaggcccctagactgctgatttgtggcgc ctcttcttgggccaccggcattcccgatagattttctggcagcggctc cggcaccgacttcaccctgacaatcagcagactggaacccgagga cttcgccgtgtactactgccagcagtacagcagcagccccatcacct ttggccagggcacaagactggaaatcaagcgg | |
| XMVA14 | VL_{anti-ANG-2}-G₄S-VH_{anti-VEGF-A}-(G₄S)₃-VL_{anti-VEGF-A}-G₄S-VH_{anti-ANG-2} | EIVLTQSPGTLSLSPGERAT LSCRASQSITGSYLAWYQ QKPGQAPRLLICGASSWA TGIPDRFSGSGSGTDFTLTI SRLEPEDFAVYYCQQYSSS PITFGQGTRLEIKRGGGGS EVQLVESGGGLVQPGGSL RLSCAASGFTISDYWIHW VRQAPGKGLEWVAGITPA GGYTYYADSVKGRFTISA DTSKNTAYLQMNSLRAED TAVYYCARFVFFLPYAMD YWGQGTLVTVSSGGGGS GGGGSGGGGSDIQMTQSP SSLSASVGDRVTITCRASQ DVSTAVAWYQQKPGKAP KLLIYSASFLYSGVPSRFS GSGSGTDFTLTISSLQPEDF ATYYCQQGYGNPFTFGQG TKVEIKRGGGGSQVQLVE SGGGVVQPGRSLRLSCAA SGFTFTNYGMHWGRQAP GKGLEWVAVISHDGNNKY YVDSVKGRFTISRDNSKN TLYLQMNSLRAEDTAVYY CAREGIDFWSGLNWFDP WGQGTLVTVSSA | SEQ ID NO: 51 | gagatcgtgctgacacagagccctggcacactgtcactgtctccag gcgaaagagccacactgagctgtagagccagccagagcatcaccg gctcttacctggcttggtatcagcagaagcctggacaggcccctaga ctgctgatttgtggcgcctcttcttgggccaccggcattcccgatagat tttctggcagcggctccggcaccgacttcaccctgacaatcagcaga ctggaacccgaggacttcgccgtgtactactgccagcagtacagca gcagccccatcacctttggccagggcacaagactggaaatcaagc ggggcggagggggctctgaggtgcagctggtggagagcggagg aggactggtgcagccaggaggctccctgcggctgtcttgcgccgcc agcggctttaccatctccgactactggattcactgggtgagacaggc acctggcaagggactggagtgggtggcaggaatcaccccagcag gaggctacacatactatgccgacagcgtgaagggccggttcaccat ctccgccgatacctctaagaacacagcctatctgcagatgaactccct gcgggccgaggacacagccgtgtactattgcgccagattcgtgttct tcctgccatacgccatggattattggggccagggcaccctggtgaca gtgagctccggtggaggtggctccggtggcggtggcagcggcggt ggcggctctgatattcagatgacacagtccccatctagcctgtctgcc agcgtgggcgacagggtgaccatcacatgtagagcaagccaggac gtgagcaccgcagtggcatggtaccagcagaagcctggcaaggcc ccaaagctgctgatctactccgcctctttcctgtattctggcgtgccaa gcaggtttagcgggtccggatctggaaccgacttcaccctgacaatc tcctctctgcagcctgaggattttgccacatactattgccagcagggct atggcaatccattcacctttggccagggcacaaaggtggagatcaa gaggggcggagggggctctcaggtgcagctggttgaatctggtgg cggagttgtgcagcctggcagaagcctgagactgtcttgtgccgcc agcggcttcaccttcaccaactatggaatgcactggggcagacagg cccctggcaaaggacttgaatgggtcgccgtgatcagccacgacg gcaacaacaagtactacgtggacagcgtgaagggcagattcaccat cagccgggacaacagcaagaacaccctgtacctgcagatgaacag cctgagagccgaggacaccgccgtgtactattgtgccagagaggg catcgacttttggagcggcctgaattggttcgacccttggggacagg gcaccctggttacagtttcttctgcc | SEQ ID NO: 59 |
| | CD5-sp-VL_{anti-ANG-2}-G₄S-VH_{anti-VEGF-A}-(G4S)3-VL_{anti-VEGF-A}-G₄S-VH_{anti-ANG-2} | MPMGSLQPLATLYLLGML VASCLGEIVLTQSPGTLSLS PGERATLSCRASQSITGSY LAWYQQKPGQAPRLLICG ASSWATGIPDRFSGSGSGT DFTLTISRLEPEDFAVYYC QQYSSSPITFGQGTRLEIK RGGGGSEVQLVESGGGLV QPGGSLRLSCAASGFTISD YWIHWVRQAPGKGLEWV AGITPAGGYTYYADSVKG RFTISADTSKNTAYLQMNS LRAEDTAVYYCARFVFFL PYAMDYWGQGTLVTVSS GGGGSGGGGSGGGGSDIQ MTQSPSSLSASVGDRVTIT CRASQDVSTAVAWYQQKP GKAPKLLIYSASFLYSGVP SRFSGSGSGTDFTLTISSLQ PEDFATYYCQQGYGNPFT FGQGTKVEIKRGGGGSQV | SEQ ID NO: 52 | atgccaatggggtccctacaacccctggcaacactatatctgctaggt atgctcgtggcctcctgtttaggagagatcgtgctgacacagagccc tggcacactgtcactgtctccaggcgaaagagccacactgagctgta gagccagccagagcatcaccggctcttacctggcttggtatcagca gaagcctggacaggcccctagactgctgatttgtggcgcctcttcttg ggccaccggcattcccgatagattttctggcagcggctccggcacc gacttcaccctgacaatcagcagactggaacccgaggacttcgccg tgtactactgccagcagtacagcagcagccccatcacctttggccag ggcacaagactggaaatcaagcggggcggagggggctctgaggt gcagctggtggagagcggaggaggactggtgcagccaggaggct ccctgcggctgtcttgcgccgccagcggctttaccatctccgactact ggattcactgggtgagacaggcacctggcaagggactggagtggg tggcaggaatcaccccagcaggaggctacacatactatgccgaca gcgtgaagggccggttcaccatctccgccgatacctctaagaacac agcctatctgcagatgaactccctgcgggccgaggacacagccgtg tactattgcgccagattcgtgttcttcctgccatacgccatggattattg gggccagggcaccctggtgacagtgagctccggtggaggtggctc cggtggcggtggcagcggcggtggcggctctgatattcagatgaca cagtccccatctagcctgtctgccagcgtgggcgacagggtgacca tcacatgtagagcaagccaggacgtgagcaccgcagtggcatggt accagcagaagcctggcaaggccccaaagctgctgatctactccgc | SEQ ID NO: 60 |
| | | QLVESGGGVVQPGRSLRL SCAASGFTFTNYGMHWG RQAPGKGLEWVAVISHDG NNKYYVDSVKGRFTISRD NSKNTLYLQMNSLRAEDT AVYYCAREGIDFWSGLN WFDPWGQGTLVTVSSA | | ctctttcctgtattctggcgtgccaagcaggtttagcgggtccggatct ggaaccgacttcaccctgacaatctcctctctgcagcctgaggatttt gccacatactattgccagcagggctatggcaatccattcacctttggc cagggcacaaaggtggagatcaagaggggcggagggggctctca ggtgcagctggttgaatctggtggcggagttgtgcagcctggcaga agcctgagactgtcttgtgccgccagcggcttcaccttcaccaactat ggaatgcactggggcagacaggcccctggcaaaggacttgaatgg gtcgccgtgatcagccacgacggcaacaacaagtactacgtggac agcgtgaagggcagattcaccatcagccgggacaacagcaagaac accctgtacctgcagatgaacagcctgagagccgaggacaccgcc gtgtactattgtgccagagagggcatcgacttttggagcggcctgaat tggttcgacccttggggacagggcaccctggttacagtttcttctgcc | |
| XMVA15 | VL_{anti-VEGF-A}-G₄S - VH_{anti-ANG-2}-(G₄S)₃-VL_{anti-ANG-2}-G₄S -VH_{anti-VEGF-A} | DIQMTQSPSSLSASVGDRV TITCRASQDVSTAVAWYQ QKPGKAPKLLIYSASFLYS GVPSRFSGSGSGTDFTLTIS SLQPEDFATYYCQQGYGN PFTFGQGTKVEIKRGGGG SQVQLVESGGGVVQPGRS LRLSCAASGFTFTNYGMH WGRQAPGKGLEWVAVISH DGNNKYYVDSVKGRFTIS RDNSKNTLYLQMNSLRAE DTAVYYCAREGIDFWSGL NWFDPWGQGTLVTVSSA GGGGSGGGGSGGGGSEIV LTQSPGTLSLSPGERATLS CRASQSITGSYLAWYQQK PGQAPRLLICGASSWATGI PDRFSGSGSGTDFTLTISRL EPEDFAVYYCQQYSSSPIT FGQGTRLEIKRGGGGSEV QLVESGGGLVQPGGSLRL SCAASGFTISDYWIHWVR QAPGKGLEWVAGITPAGG YTYYADSVKGRFTISADTS KNTAYLQMNSLRAEDTAV YYCARFVFFLPYAMDYW GQGTLVTVSS | SEQ ID NO: 53 | gatattcagatgacacagtccccatctagcctgtctgccagcgtggg cgacagggtgaccatcacatgtagagcaagccaggacgtgagcac cgcagtggcatggtaccagcagaagcctggcaaggccccaaagct gctgatctactccgcctctttcctgtattctggcgtgccaagcaggttta gcgggtccggatctggaaccgacttcaccctgacaatctcctctctg cagcctgaggattttgccacatactattgccagcagggctatggcaat ccattcacctttggccagggcacaaaggtggagatcaagaggggc ggagggggctctcaggtgcagctggttgaatctggtggcggagttg tgcagcctggcagaagcctgagactgtcttgtgccgccagcggcttc accttcaccaactatggaatgcactggggcagacaggcccctggca aaggacttgaatgggtcgccgtgatcagccacgacggcaacaaca agtactacgtggacagcgtgaagggcagattcaccatcagccggg acaacagcaagaacaccctgtacctgcagatgaacagcctgagag ccgaggacaccgccgtgtactattgtgccagagagggcatcgacttt tggagcggcctgaattggttcgacccttggggacagggcaccctgg ttacagtttcttctgccggtggaggtggctccggtggcggtggcagc ggcggtggcggctctgagatcgtgctgacacagagccctggcaca ctgtcactgtctccaggcgaaagagccacactgagctgtagagcca gccagagcatcaccggctcttacctggcttggtatcagcagaagcct ggacaggcccctagactgctgatttgtggcgcctcttcttgggccac cggcattcccgatagattttctggcagcggctccggcaccgacttca ccctgacaatcagcagactggaacccgaggacttcgccgtgtacta ctgccagcagtacagcagcagccccatcacctttggccagggcaca agactggaaatcaagcggggcggagggggctctgaggtgcagct ggtggagagcggaggaggactggtgcagccaggaggctccctgc ggctgtcttgcgccgccagcggctttaccatctccgactactggattc actgggtgagacaggcacctggcaagggactggagtgggtggca ggaatcaccccagcaggaggctacacatactatgccgacagcgtg aagggccggttcaccatctccgccgatacctctaagaacacagccta tctgcagatgaactccctgcgggccgaggacacagccgtgtactatt gcgccagattcgtgttcttcctgccatacgccatggattattggggcc agggcaccctggtgacagtgagctcc | SEQ ID NO: 61 |
| | CD5-sp-VL_{anti-VEGF-A}-G₄S - VH_{anti-ANG-2}-(G₄S)₃-VL_{anti-ANG-2}-G₄S -VH_{anti-VEGF-A} | MPMGSLQPLATLYLLGML VASCLGDIQMTQSPSSLSA SVGDRVTITCRASQDVSTA VAWYQQKPGKAPKLLIYS ASFLYSGVPSRFSGSGSGT DFTLTISSLQPEDFATYYC QQGYGNPFTFGQGTKVEI KRGGGGSQVQLVESGGG VVQPGRSLRLSCAASGFT FTNYGMHWGRQAPGKGL EWVAVISHDGNNKYYVDS VKGRFTISRDNSKNTLYLQ MNSLRAEDTAVYYCAREG IDFWSGLNWFDPWGQGT LVTVSSAGGGGSGGGGSG GGGSEIVLTQSPGTLSLSP GERATLSCRASQSITGSYL AWYQQKPGQAPRLLICGA SSWATGIPDRFSGSGSGTD FTLTISRLEPEDFAVYYCQ QYSSSPITFGQGTRLEIKR GGGGSEVQLVESGGGLVQ PGGSLRLSCAASGFTISDY WIHWVRQAPGKGLEWVA GITPAGGYTYYADSVKGR FTISADTSKNTAYLQMNSL | SEQ ID NO: 54 | atgccaatggggtccctacaacccctggcaacactatatctgctaggt atgctcgtggcctcctgtttaggagatattcagatgacacagtccccat ctagcctgtctgccagcgtgggcgacagggtgaccatcacatgtag agcaagccaggacgtgagcaccgcagtggcatggtaccagcaga agcctggcaaggccccaaagctgctgatctactccgcctctttcctgt attctggcgtgccaagcaggtttagcgggtccggatctggaaccga cttcaccctgacaatctcctctctgcagcctgaggattttgccacatac tattgccagcagggctatggcaatccattcacctttggccagggcac aaaggtggagatcaagaggggcggagggggctctcaggtgcagc tggttgaatctggtggcggagttgtgcagcctggcagaagcctgag actgtcttgtgccgccagcggcttcaccttcaccaactatggaatgca ctggggcagacaggcccctggcaaaggacttgaatgggtcgccgt gatcagccacgacggcaacaacaagtactacgtggacagcgtgaa gggcagattcaccatcagccgggacaacagcaagaacaccctgta cctgcagatgaacagcctgagagccgaggacaccgccgtgtactat tgtgccagagagggcatcgacttttggagcggcctgaattggttcga cccttggggacagggcaccctggttacagtttcttctgccggtggag gtggctccggtggcggtggcagcggcggtggcggctctgagatcg tgctgacacagagccctggcacactgtcactgtctccaggcgaaag agccacactgagctgtagagccagccagagcatcaccggctcttac ctggcttggtatcagcagaagcctggacaggcccctagactgctgat ttgtggcgcctcttcttgggccaccggcattcccgatagattttctggc agcggctccggcaccgacttcaccctgacaatcagcagactggaac ccgaggacttcgccgtgtactactgccagcagtacagcagcagccc catcacctttggccagggcacaagactggaaatcaagcggggcgg agggggctctgaggtgcagctggtggagagcggaggaggactgg | SEQ ID NO: 62 |
| | | RAEDTAVYYCARFVFFLP YAMDYWGQGTLVTVSS | | tgcagccaggaggctccctgcggctgtcttgcgccgccagcggctt taccatctccgactactggattcactgggtgagacaggcacctggca agggactggagtgggtggcaggaatcaccccagcaggaggctac acatactatgccgacagcgtgaagggccggttcaccatctccgccg atacctctaagaacacagcctatctgcagatgaactccctgcgggcc gaggacacagccgtgtactattgcgccagattcgtgttcttcctgcca tacgccatggattattggggccagggcaccctggtgacagtgagct cc | |

### Example 6. Preparation and identification of recombinant AAV virus

The XMVA09 construct and ssAAV plasmid were double digested with BamH I/EcoR V, and ssAAV-XMVA09 vector was constructed by conventional molecular biology operations such as ligation, transformation, cloning screening and identification, and the vector information is shown in FIG. 2B. High-quality plasmid DNA was obtained for later use by using an endotoxin-free plasmid extraction kit (MN), and recombinant AAV virus was prepared by using a three-plasmid packaging system, a helper plasmid (phelper), a Cap and Rep protein expression plasmid of AAV, a plasmid expressing the target gene (ssAAV-XMVA09) in a mass ratio of 2 : 1: 1 were used to form a transfection complex with PEI transfection promoter, and were transfected into HEK293T cells to conduct AAV-XMVA09 packaging. The supernatant was collected twice at day 3 and day 7 after transfection to obtain AAV particles containing the target gene. Density gradient centrifugation (Beckman's ultracentrifuge) was performed with different gradients of iodoxanol (15%, 25%, 40% and 60%) to obtain purified AAV viraus. The AAV quality was identified by transmission electron microscopy and the AAV virus titer was quantified by qPCR.

### Example 7. Inhibitory effect of XMVA09 on laser-induced CNV in wAMD model mice

The animal model of CNV induced by retinal high-energy laser photocoagulation is a commonly used animal model at home and abroad, and is a standard animal model in most treatment evaluation experiments at present. It selectively destroys the photoreceptor outer segment disc membrane, Bruch membrane, retinal pigment epithelium and part of the anterior choroidal capillary network are selectively damaged, followed by a damage repair reaction, including invasion and growth of fibroblast, retinal pigment epithelial cell and vascular endothelial cell, and finally, neovascularization is formed in a photocoagulation area.

Thirty SPF grade 6~8 weeks male C57 BL/6J mice (purchased from Beijing Vital River Laboratories Experimental Animal Technology Co., Ltd.), weighing 22-25 g were used, and the specific administration scheme is shown in Table 4 below:

**Table 4. Administration scheme**

| group | medicine administered | medicine concentration (vg/mL) | administratio n volume (µL) | administratio n dosage (vg/eye) | administratio n route | number of animals |
|---|---|---|---|---|---|---|
| control | PBS | - | 2.5 | - | intravitreal injection | 15 |
| XMVA09 | AAV-XMVA09 | 2.5 × 10¹² | 2.5 | 6.25 × 10⁹ | intravitreal injection | 15 |

Since the expression of the target gene reached a stable level after a certain period of time after AAV injection, intravitreal injection was performed on day 1, and binocular funduslaser photocoagulation was performed on the successfully injected animals on day 22 to induce CNV model.

Establishment of mouse CNV model: The pupils of both eyes of the mice were dilated with 1-2 drops of topicamide eye drops, and 5% chloral hydrate was injected intramuscularly for anesthesia. After anesthesia, carbomer eye drops were dropped in both eyes, a fundus laser scope was placed, and photocoagulation was performed around the optic papilla at a distance of about 1.5-2 PD from the optic disc avoiding blood vessels. Laser parameters were as follows: wavelength 532 nm, energy 80 mW, spot size 50 µm, exposure time 100 ms, and erythromycin eye ointement were applied to both eyes of the animal after photocoagulation.

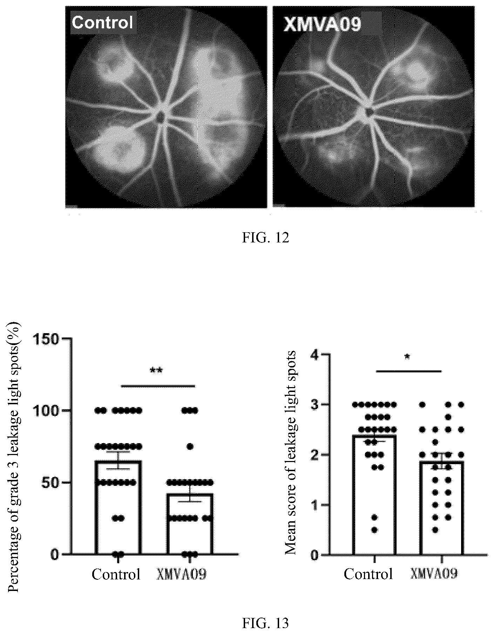

On day 29, fluorescein fundus angiography (FFA) detection was performed on mice: fluorescein sodium injection (15 mg/mL, 10 mL/kg) was intraperitoneally injected, several clear pictures of both eyes were collected at early (within 1.5 minutes) and late (after 3 minutes) stages after the fluorescein sodium injection, the fluorescence leakage degree of the effective light spots were rated, and the percentage of grade 3 leakage light spots and the mean score of leakage light spots were calculated. The results are shown in FIG. 12 and FIG. 13. In the laser-induced wAMD model mice, the formation of CNV was significantly inhibited after a single intravitreal injection of AAV-XMVA09.

Effective light spot refers to a light spot that has no severe retinal hemorrhage nearby and can be completely displayed in the FFA. The grading standards of spots fluorescence leakage are as follows: grade 0 (no fluorescence leakage), grade 1 (mild fluorescence leakage, with a leakage area of 1-50% of the laser spot size), grade 2 (moderate fluorescence leakage, with a leakage area of 50 -100% of the laser spot size), grade 3 (severe fluorescence leakage, with a leakage area larger than the laser spot size).

Percentage (%) of light spots of each grade = total number of light spots of the corresponding grade ÷ total number of 4 types of light spots × 100%.

Mean score of leakage light spot = [(number of grade 0 light spots × 0) + (number of grade 1 light spots× 1) + (number of grade 2 light spots × 2) + (number of grade 3 light spots × 3)] ÷ total number of 4 types of light spots.

### Example 8. Inhibitory effect of XMVA09 on CNV in laser-induced wAMD model rhesus monkeys

Seven male rhesus monkeys (purchased from Ya'an Primed Bio-tech Co., Ltd.) with a common grade (qualified in quarantine before test) of 5-6 years old were used, and the specific administration scheme is shown in table 5 below:

**Table 5. Administration scheme**

| group | medicine administered | medicine concentration (vg/mL) | administrati on volume (µL) | administratio n dosage (vg/eye) | administratio n route | number of animals |
|---|---|---|---|---|---|---|
| control | PBS | - | 50 | - | intravitreal injection | 3 |
| XMVA09 | AAV-XMVA09 | 1.94×10¹² | 50 | 9.7×10¹⁰ | intravitreal injection | 4 |

XMVA09 Group: AAV-XMVA09 (50 µL/eye) was administered by intravitreal injection in both eyes on day 21 before laser modeling, and CNV was induced in both eyes by fundus laser on days 0.

Control group: CNV was induced in both eyes by fundus laser on day 0, and PBS (50 µL/eye) was administered by intravitreal injection in both eyes on day 21.

All animals were examined by optical coherence tomography (OCT) prior to laser modeling and fundus photography (FP), FFA and OCT examinations were performed on day 19 and day 77 of laser modeling; and the number, leakage area and retinal thickness of grade IV light spots were used as main efficacy evaluation indicators. Establishment of the rhesus monkey CNV model: animals were anesthetized by intramuscular injection of ketamine hydrochloride (20 mg/kg) and dexmedetomidine hydrochloride (0.03 mg/kg), and the pupils of both eyes were dilated with Midori (compound tropicamide eye drops) drops. After the pupils of both eyes were larger than 6 mm, the head of the animal was fixed in front of an ophthalmic laser photocoagulator, and after the retinal structure was peculated through a fundus scope, laser photocoagulating was carried out at an optic disc distance around the center of the macula, with 9 points in each eye. The laser parameters were: wavelength 532 nm, energy 650 mW-700 mW, spot size 50 µm, exposure time 0.1 second. Fluorescein leakage area was measured by FP and FFA, and the specific examination method was as follows: (1) animals were anesthetized by intramuscular injection of ketamine hydrochloride (20 mg/kg) and dexmedetomidine hydrochloride (0.03 mg/kg). (2) The pupils of both eyes were dilated with 1-2 drops of compound tropicamide eye drops. (3) The pupil of the animal was observed after pupil dilation, if the pupil was greater than 6 mm under light, a colour photograph of the fundus centered on the macula was taken by a fundus camera. (4) After that, a 0.075 mL/kg dose of 10% fluorescein sodium injection (Fluorescite, Alcon) was rapidly injected from the saphenous vein of the lower limb. FFA photographs centered on the macula were taken by fundus cameras at early (within 1 minute), medium (5 minutes) and late (10 minutes) stages after fluorescein injection. (6) The fluorescein leakage area of the grade IV spot in the late stage (10 min) of FFA photo was automatically measured by Image J software (version 1.53e, NIH, Wayne, Raband, USA) as an evaluation indicator of the efficacy of FFA. The fluorescein leakage grading standard of the laser spot is: grade I (the light spot has no high fluorescence), grade II (the light spot has high fluorescence without leakage), grade III (the light spot has high fluorescence with slight fluorescein leakage, but leakage does not exceed the edge of the light spot), grade IV (the light spot has high fluorescence with significant fluorescein leakage, and leakage exceeds the edge of the light spot).

Retinal thickness changes were measured by OCT, and the specific examination method is as follows: (1) after completing the FP and FFA examination, the animals were placed in front of an OCT instrument, and the animal's eyes were adjusted to look directly at the scanning lens. (2) Multi-layer linear scanning was performed by adopting a follow-up mode centered on the macula, with the scanning area covered all laser points. (3) A layer with the most obvious retinal thickness change and clear retinal boundary after modeling was selected, and the maximum retinal thickness value of the region was measured by using measurement software carried by the instrument to serve as an OCT efficacy evaluation indicator.

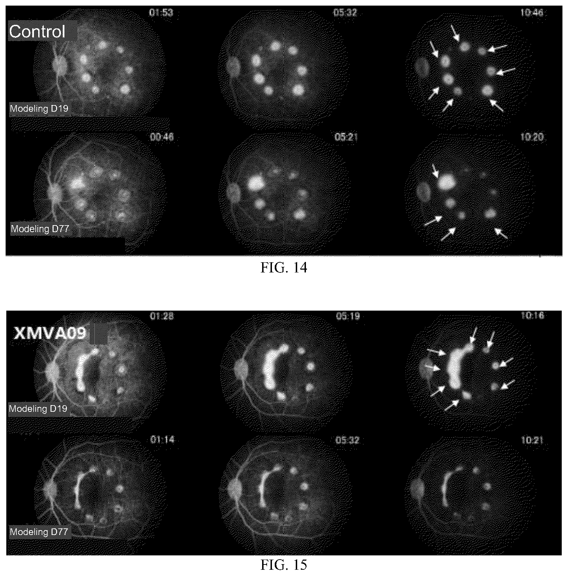

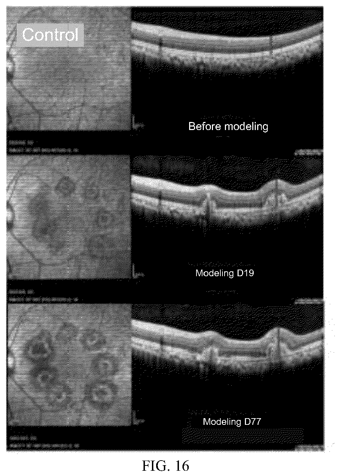

The results are shown in FIG. 14, FIG. 15, FIG. 16 and FIG. 17. On the day 19 and day 77 of the laser modeling in each group of animals, FFA examination showed that different fluorescence leakage performance at the laser spots in the fundus. Compared with the control group, a single intravitreal injection of AAV-XMVA09 has an obvious improvement effect on the number, leakage area and retinal thickness of grade IV light spots of CNV in laser-induced wAMD rhesus monkey model.

### Example 9. High glucose-induced increase in HRMECs permeability model

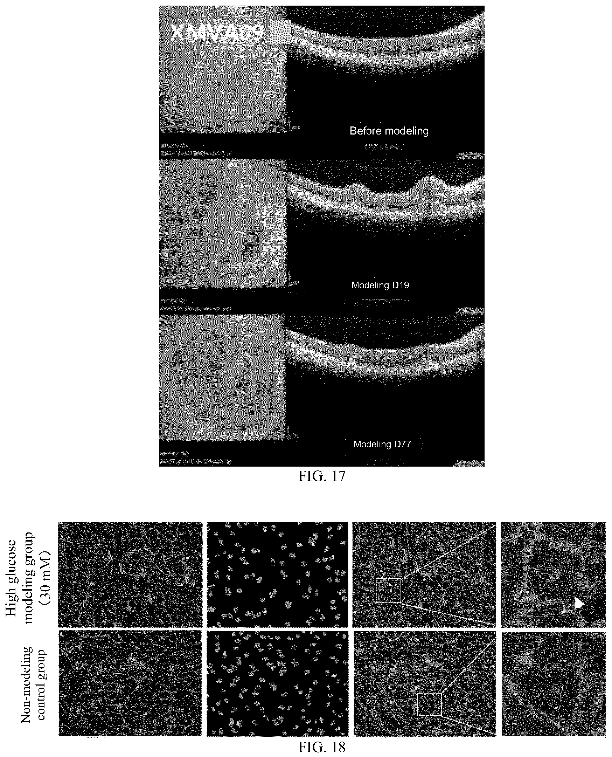

The 24-well plate was pre-treated with 0.1% gelatin at 37°C for 1-2 hours and then dried for several minutes ; then the HRMECs were resuspended in complete culture medium of ECM (Sciencell) containing 10% FBS + 1% PS + 1% ECGS, and spread evenly in the well plate according to the standard of 3 × 10⁵ / well; on the next day, starvation treatment was initiated with a low-serum culture medium containing 0.5% FBS for 7 hours; then the HRMECs were exposed to 30 mM high glucose environment overnight; the control group was not treated with high glucose; on the next day, immunofluorescence co-staining of cells for membrane protein with VE-cadherin and nuclear with DAPI was performed, for fluorescence microscope observation and photography. The results are shown in FIG. 18. The cell membrane continuity of HRMECs treated with high glucose was disrupted, resulting in intercellular enlargement and increased permeability.

Steps of immunofluorescence staining : discarding the supernatant of the culture medium so that the remaining volume was 200 µL, and adding 200 µL of 4% PFA to start the gradient fixation for 20 minutes at room temperature; after discarding the supernatant, directly adding 200 µL of PFA, and continuing to fix at room temperature for 10 minutes; after discarding the fixation solution, washing with PBS for 3 times, each time for 5 minutes; diluting VE-cadherin (Santa Cruz Biotechnology Inc) at 1:150 with PBS, then adding 300 µL/well, and incubating at room temperature for 2 hours; discarding the supernatant, washing with PBS for 3 times, each time for 5 minutes; diluting the Donkey anti-Mouse IgG(H+L) Highly Cross-Adsorbed Secondary Antibody, Alexa Fluor^{™} 488 (Invitrogen) at 1:200 with PBS, adding 200 µL per well, and incubating at 37°C for 1 hour; discarding the supernatant, washing with PBS for 3 times, each time for 5 minutes; and finally adding DAPI-containing mounting agent (Beyotime), 100 µL/well, and observing and photographing under a fluorescence microscope.

### Example 10. Determining the regulatory effect of XMVA09 protein on the permeability of HRMECs based on VE-cadherin indicator

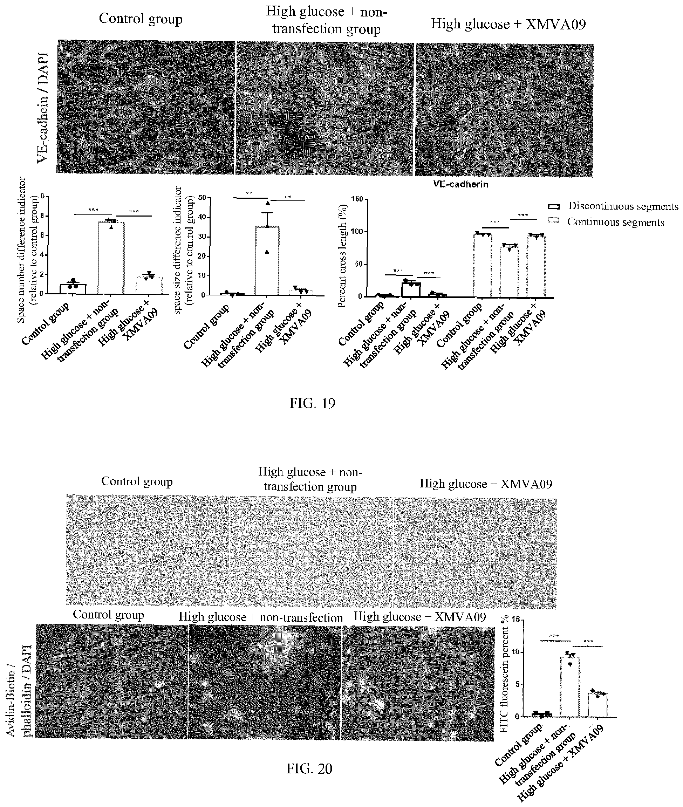

The 24-well plate was pre-coated with 0.1% gelatin at 4°C overnight or at 37°C for 2 hours; the coating solution was discarded, and HRMECs cells were inoculated 3 × 10⁵/well; on the next day, when the monolayer cells reached a certain confluence, starvation treatment was initiated with a low-serum medium containing 0.5% FBS for 7 hours; the supernatant of untransfected HEK293T cells and the supernatant of HEK293T cells transfected with ssAAV-XMVA09 plasmid were co-incubated with high glucose (30 mM glucose), respectively. HRMECs were treated overnight, and the control group was a non-modeling group without high glucose treatment. Immunofluorescence staining of VE-cadherin was performed, and the detailed steps were as in Example 9, and photographic analysis was performed under a fluorescence microscope. The results are shown in FIG. 19. The protein expressed by ssAAV-XMVA09 has a significant inhibitory effect on the increase of permeability of HRMECs after high glucose treatment.

### Example 11. Determining the regulatory effect of XMVA09 protein on the permeability of HRMECs based on biotin-avidin system indicator

Gelatin was treated with EZ-Link^{™} NHS-LC-LC-biotin to obtain biotinylated gelatin; the biotinylated gelatin was used to coat a cell culture plate at 4°C overnight or 37°C for 2 hours; HRMECs were directly inoculated in the culture plate, and the inoculation amount of cells was 3 × 10⁵/well; on the next day, starvation treatment was first initiated with a low-serum medium containing 0.5% FBS for 7 hours; then the supernatant of untransfected HEK293T cells and the supernatant of HEK293T cells transfected with ssAAV-XMVA09 plasmid were co-incubated with high glucose (30 mM glucose), respectively, HRMECs were treated for 17 hours, and the control group was a non-modeling group without high glucose treatment. After saining with FITC-avidin and fixation with 4% PFA, staining with AF594-phaloidin (Invitrogen A12381) was started, then an anti-fluorescence quencher containing DAPI was added, and photographical analysis was performed under a fluorescence microscope. The results are shown in FIG. 20. The protein expressed by ssAAV-XMVA09 has a significant inhibitory effect on the increase of permeability of HRMECs after high glucose treatment.

### Example 12. Construction of Diabetic Mouse Model

Drug induction is one of the current methods for establishing diabetic animal models, which can better simulate clinical pathological manifestations and clinical characteristics of diabetes. Streptozotocin (STZ) is the most commonly used medicament for inducing diabetes, and it mainly causes hyperglycemia by destroying pancreatic beta cells, which in turn causes pericapillary cell loss, vascular layer thinning and blood-retinal barrier destruction, further causes vessel leakage, and it is a good model for studying DME.

### Establishment of Diabetic Mouse Model:

Eighty SPF grade 6 ~ 8 weeks male C57 BL/6J mice (purchased from Zhejiang Vital River Laboratories Experimental Animal Technology Co., Ltd.), weighing 20-25g, were randomly divided into blank group, DME model control group, and DME modeling group. Before modeling, fasting blood glucose and body weight of three groups of mice were measured; after fasting for 6 hours (with free access to water), blood was collected at the tail tip for blood glucose measurement and the mice were weighed, on day 0 of modeling. Modeling was started on day 1, and mice were fasted (with free access to water) for 6 hours. The Blank group was not treated, the DME model control group recieved a single intraperitoneal injection of 50 mM sodium citrate solution (Sigma) 60 mg / kg for 5 continuous days, and the DME model group recieved a single intraperitoneal injection of STZ (Sigma) 60 mg/kg for 5 continuous days. The mice were free to eat and drink, blood glucose and body weight were measured and recorded regularly, and the modeling lasted for 135 days.

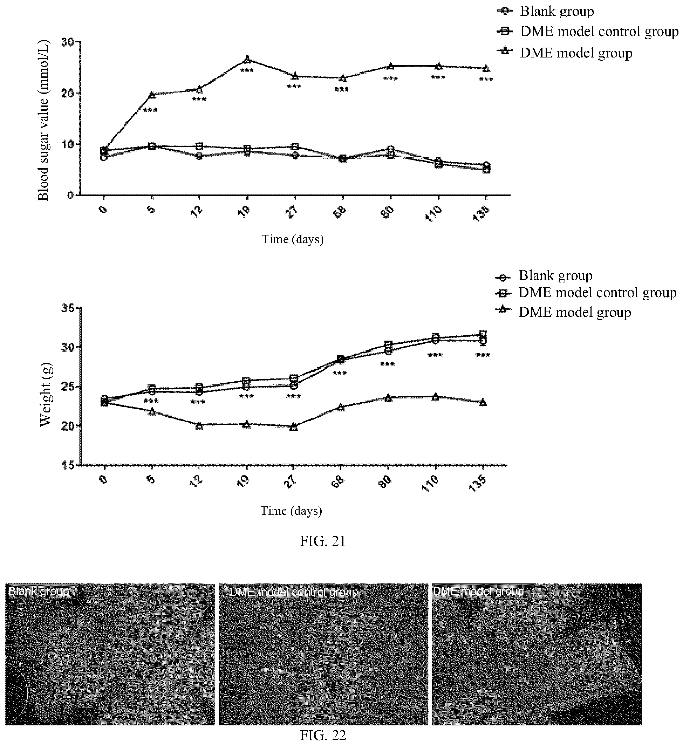

Results are shown in FIG. 21. On day 19 after modeling, the fasting blood glucose values of mice were higher than 16.7 mmol/L for about three weeks, indicating the diabetic mice model was successfully constucted.

### Example 13. Phenotypic validation of DME in diabetic mouse model

On day 81 after modeling, a portion of mice in each of the three groups of mice in Example 12 were injected with Evans Blue by tail vein injection, the retinas were dissected and separated, and vascular leakage was observed under a fluorescence microscope. The specific operations are as follows: 50 mg/mL of Evans blue (Sigma) was injected 50 µL/mouse into the tail vein; after 1.5 hours of blood circulation, the mice were euthanized, both eyes were enucleated and fixed for 45 minutes in 4% paraformaldehyde (Biosharp), the retina of the eyeball was peeled off and spread, and observed and photographed under a fluorescence microscope.

Results are shown in FIG. 22. The retinal vessel leakage of the DME model group was obviously increased compared with the DME model control group and the Blank group, there was no obvious difference between the retinas of the DME model control group and the Blank group, and the diabetic mouse models had a DME phenotype.

### Example 14. Inhibitory effect of XMVA09 on retinal vascular leakage in diabetic mouse model

The DME model mice in Example 12 were administered by injection on day 95 after molding, and the specific administration scheme is shown in Table 6 below:

**Table 6. Administration Scheme**

| group | medicine administered | medicine concentration (vg/mL) | administratio n volume (µL) | administratio n dosage (vg/eve) | administratio n route | number of animals |
|---|---|---|---|---|---|---|
| Control | PBS | - | 2.5 | - | intravitreal injection | 6 |
| XMVA09 | AAV-XMVA09 | 2.5 × 10¹² | 2.5 | 6.25 × 10⁹ | intravitreal injection | 6 |

On day 29 after injection, mice in the above XMVA09 group, Control group and mice in the DME model control group in Example 12 were injected with Evans blue by tail vein injection and dissect the retina to observe blood vessel leakage under fluorescence microscope.

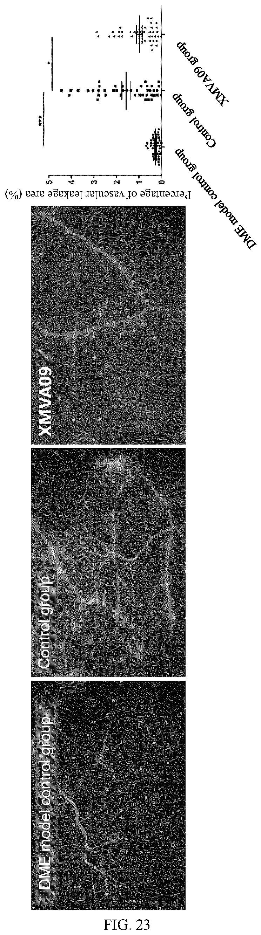

The results are shown in FIG. 23. The percentage of retinal vascular leakage in the DME model control group is significantly lower than that in the Control group, and the percentage of retinal vascular leakage in the XMVA09 group is significantly lower than that in the Control group, which indicates that a single intravitreal injection of AAV-XMVA09 has a significant inhibition effect on retinal vascular leakage in the diabetic mouse model.

### Statistical Analysis

GraphPad Prism 8.0 software was used for data processing and statistical analysis. The statistical level was set to 5% or p≤0.05, and the average number and standard error (Mean ± SEM) of each analysis indicator were calculated, where p≤0.05 means the difference is statistically significant.

## Claims

1. A bispecific antibody comprising anti-VEGF-A and anti-ANG-2 binding domains, the heavy chain variable regions (VH) and light chain variable regions (VL) of the bispecific antibody are arranged from N-terminus to C-terminus in the following order:
1) VH_{anti-VEGF-A}-VL_{anti-VEGF-A}-VH_{anti-ANG-2}-VL_{anti-ANG-2};
2) VL_{anti-ANG-2}-VH_{anti-VEGF-A}-VL_{anti-VEGF-A}-VH_{anti-ANG-2}; or
3) VL_{anti-VEGF-A}-VH_{anti-ANG-2}-VL_{anti-ANG-2}-VH_{anti-VEGF-A};
wherein the VH_{anti-VEGF-A} comprises a CDR1 as depicted in SEQ ID NO: 1, a CDR2 as depicted in SEQ ID NO: 2, and a CDR3 as depicted in SEQ ID NO: 3;
the VL_{anti-VEGF-A} comprises a CDR1 as depicted in SEQ ID NO: 4, a CDR2 as depicted in SEQ ID NO: 5, and a CDR3 as depicted in SEQ ID NO: 6 or SEQ ID NO: 47;
the VH_{anti-ANG-2} comprises a CDR1 as depicted in SEQ ID NO: 7, a CDR2 as depicted in SEQ ID NO: 8, and a CDR3 as depicted in SEQ ID NO: 9;
the VL_{anti-ANG-2} comprises a CDR1 as depicted in SEQ ID NO: 10, a CDR2 as depicted in SEQ ID NO: 11, and a CDR3 as depicted in SEQ ID NO: 12.

2. The bispecific antibody of claim 1, wherein
the VH_{anti-VEGF-A} comprises a sequence as depicted in SEQ ID NO: 13 or a sequence that is 70%, 80%, 90%, 95% or 99% identical to the sequence of SEQ ID NO: 13;
the VL_{anti-VEGF-A} comprises a sequence as depicted in SEQ ID NO: 14 or a sequence that is 70%, 80%, 90%, 95% or 99% identical to the sequence of SEQ ID NO: 14 or SEQ ID NO: 48;
the VH_{anti-ANG-2} comprises a sequence as depicted in SEQ ID NO: 15 or a sequence that is 70%, 80%, 90%, 95% or 99% identical to the sequence of SEQ ID NO: 15;
the VL_{anti-ANG-2} comprises a sequence as depicted in SEQ ID NO: 16 or a sequence that is 70%, 80%, 90%, 95% or 99% identical to the sequence of SEQ ID NO: 16.

3. The bispecific antibody of claim 1, wherein the antibody comprises a sequence as depicted in SEQ ID NO: 17, 18, 19, 49, 51 or 53 or a sequence that is 70%, 80%, 90%, 95% or 99% identical to the sequence of SEQ ID NO: 17, 18, 19, 49, 51 or 53.

4. The bispecific antibody of claim 1, wherein the N-terminus of the bispecific antibody comprises a signal peptide sequence or a tag sequence, preferably the signal peptide sequence is a CD5-sp signal peptide as depicted in SEQ ID NO: 23.

5. The bispecific antibody of claim 4, wherein the antibody comprises a sequence as depicted in SEQ ID NO: 20, 21, 22, 50, 52 or 54 or a sequence that is 70%, 80%, 90%, 95% or 99% identical to the sequence of SEQ ID NO: 20, 21, 22, 50, 52 or 54.

6. The bispecific antibody of claim 1, wherein the heavy chain variable region (VH) and the light chain variable region (VL) are linked by (G₄S)n, wherein n = any integer between 1 and 4.

7. The bispecific antibody of claim 1, wherein the structure of the bispecific antibody is:
VH_{anti-VEGF-A} - (G₄S)₃ - VL_{anti-VEGF-A} - G₄S - VH_{anti-ANG-2} - (G₄S)₃ - VL_{anti-ANG-2};
VL_{anti-ANG-2} - G₄S - VH_{anti-VEGF-A} - (G₄S)₃ - VL_{anti-VEGF-A} - G₄S - VH_{anti-ANG-2};
VL_{anti-VEGF-A} - G₄S - VH_{anti-ANG-2} - (G₄S)₃ - VL_{anti-ANG-2} - G₄S - VH_{anti-VEGF-A};
CD5-sp - VH_{anti-VEGF-A} - (G₄S)₃ - VL_{anti-VEGF-A} - G₄S - VH_{anti-ANG-2} - (G₄S)₃ - VL_{anti-ANG-2};
CD5-sp - VL_{anti-ANG-2} - G₄S - VH_{anti-VEGF-A} - (G₄S)₃ - VL_{anti-VEGF-A} - G₄S - VH_{anti-ANG-2}; or
CD5-sp - VL_{anti-VEGF-A} - G₄S - VH_{anti-ANG-2} - (G₄S)₃ - VL_{anti-ANG-2} - G₄S - VH_{anti-VEGF-A}.

8. A nucleic acid sequence encoding the bispecific antibody of claims 1-7.

9. A vector comprising a nucleic acid sequence encoding the bispecific antibody of claims 1-7, wherein the vector is preferably an AAV virus vector.

10. The AAV virus vector of claim 9, further comprising: a 5' ITR and a 3' ITR, a promoter, a polyA sequence.

11. An AAV virus particle comprising the AAV virus vector of claim 9 or 10 and a capsid protein.

12. The AAV virus particle of claim 11, wherein the serotype of the capsid protein is AAV1, AAV2, AAV4, AAV5, AAV6, AAV7, AAV8, or AAV9.

13. A pharmaceutical composition comprising at least one of the bispecific antibody of claims 1-7, the nucleic acid sequence of claim 8, the vector of claim 9 or 10, the AAV virus particle of claim 11 or 12, and a pharmaceutically acceptable carrier.

14. Use of the bispecific antibody of claims 1-7, the nucleic acid sequence of claim 8, the vector of claim 9 or 10, the AAV virus particle of claim 11 or 12 or the pharmaceutical composition of claim 13 in the preparation of a medicament for treating or preventing cancer, intraocular neovascularization syndrome, rheumatoid arthritis, psoriasis, proliferative retinopathy, age-related macular degeneration or diabetic macular edema.

15. The use of claim 14, wherein the age-related macular degeneration is wet age-related macular degeneration.

16. The use of claim 14 or 15, wherein the medicament is administered by intravitreal, subretinal or suprachoroidal injection.
